Europäisches Patentamt

European Patent Office

Office européen des brevets

(19)

(11) EP 0 829 022 B1

(12) EUROPEAN PATENT SPECIFICATION

(45) Date of publication and mention
of the grant of the patent:
**16.01.2002 Bulletin 2002/03**

(21) Application number: **96916290.8**

(22) Date of filing: **24.05.1996**

(51) Int Cl.[7]: **G01T 1/00**, G01T 1/161,
G01T 1/164

(86) International application number:
**PCT/IT96/00107**

(87) International publication number:
**WO 96/37791 (28.11.1996 Gazette 1996/52)**

(54) **AN APPARATUS FOR SCINTIGRAPHIC ANALYSIS, PARTICULARLY A MAMMOGRAPH, WITH SUB-MILLIMETRIC SPATIAL RESOLUTION**

VORRICHTUNG ZUR SZINTIGRAPHISCHEN DARSTELLUNG, INSBESONDERE IN DER MAMMOGRAPHIE, MIT EINER RAUMLICHEN AUFLÖSUNG IM SUBMILLIMETERBEREICH

APPAREIL D'ANALYSE SCINTIGRAPHIQUE, NOTAMMENT MAMMOGRAPHE, A RESOLUTION SPATIALE SOUS-MILLIMETRIQUE

(84) Designated Contracting States:
**AT BE CH DE DK ES FI FR GB GR IE IT LI LU MC NL PT SE**

(30) Priority: **26.05.1995 IT RM950353**

(43) Date of publication of application:
**18.03.1998 Bulletin 1998/12**

(73) Proprietor: **POL.HI.TECH. S.r.l.**
**67061 Carsoli (IT)**

(72) Inventors:
• **DE NOTARISTEFANO, Francesco,**
**Pol.Hi.Tech. s.r.l.**
**I-67061 Carsoli (IT)**

• **PANI, Roberto, Pol.Hi.Tech. s.r.l.**
**I-67061 Carsoli (IT)**
• **MEONI, Massimo, Pol.Hi.Tech. s.r.l.**
**I-67061 Carsoli (IT)**
• **GRAMMATICO, Andrea, Pol.Hi.Tech. s.r.l.**
**I-67061 Carsoli (IT)**
• **SCOPINARO, Francesco, Pol.Hi.Tech. s.r.l.**
**I-67061 Carsoli (IT)**

(74) Representative: **Taliercio, Antonio et al**
**ING. BARZANO' & ZANARDO ROMA S.p.A. Via Piemonte, 26**
**00187 Roma (IT)**

(56) References cited:
**EP-A- 0 666 483**         **US-A- 5 032 728**
**US-A- 5 289 510**         **US-A- 5 461 233**

# Description

This invention generally relates to apparatuses for scintigraphic analysis and it more particularly but not exclusively concerns a scintigraphic mammograph adapted to furnish a sub-millimetric spatial resolution.

## STATE OF THE ART

The noticeable frequency of mammary tumors is presently raising the need that more and more sophisticated and frequent checks be made in order to locate any tumoral cells originating in these body organs.

Present day diagnostic methods are based upon use of a mammograph as an investigation tool: it consists of a radiographic system based upon use of X-rays emitted by an emitter tube forming the head of apparatus. The X-rays emitted from the tube pass through the breast that is compressed between a pressure member, transparent to X-rays (conventionally made of Plexiglas), arranged in upper position, where the tube is positioned, and a cassette containing a radiographic plate, arranged in lower position, opposite to the tube. Upon passing through said pressure member and the breast, the X-rays expose the radiographic plate housed in the cassette. The apparatus enables the whole head - pressure member - cassette assembly to be rotated so as to take radiographs from various directions (usually three directions: vertical, oblique and lateral direction). The so resulting mammographic image enables any existing calcification to be located, for instance nodules or microcalcifications of a possible mammary carcinoma. or simply cysts of the lactiferous ducts which could degenerate into cancer, even not always they do it. Any existing calcification, therefore, indicative or not of the real existence of a tumor is evidenced by such a mammograph. The involved spatial resolution is very high (about 1 mm). The mammary tissue has a so low density as to be completely transparent to X-rays and the sole substance absorbing such radiation is the calcium of said calcifications: in mammography, in fact, it is not possible to use synthetic contrast liquids (such as usually used in radiographic analysis of soft tissues), because they fail to reach the interested body organ. This means that, should the mammograph furnish a whatsoever indication, it is not possible to certainly establish the presence of a tumor, unless further analytical investigations are carried out, such as surgical extraction of tissue portions and subsequent biopsy thereof. All mammographic methods, therefore, can be considered up to now as very sensitive but scarcely specific methods.

## ALTERNATIVE TECHNIQUES

Various diagnostic techniques exist for analysing tumors. When such techniques are combined with other methods, such as radiography, TAC, NMR, and like, they offer noticeable possibilities to investigate the human body and to locate and quantize the existence of tumoral cells.

## a) SPECT (SINGLE PHOTON EMISSION COMPUTERIZED TOMOGRAPHY

This technique which is performed by means of an apparatus called Anger camera (or gamma-camera) provides for injecting into the body of the patient a chemical substance labelled with suitable radioisotopes which emit gamma radiations of a specific energy level. This chemical substance is selectively absorbed in the tumoral cells and emits gamma radiation amounts which are directly proportional to the radiopharmaceutical amount absorbed in the tumoral cells existing in a specific position.

The emitted gamma radiations are collimated by a suitable collimator and hit a scintillating crystal (usually a crystal of sodium iodide (NaI) having identical dimensions as the collimator, so as to be totally absorbed therefrom. The NaI crystal scintillates, thereby emitting a light amount proportional to the energy and to the amount of incident gamma rays. The light emitted by the crystal over a 360° range will be intercepted (as far as it half section facing to a direction opposite to the direction of the gamma rays is concerned) by means of a photo-multiplier system optically adhering to the crystal surface. Each photo-multiplier generates a signal proportional to the intercepted light amount. All signals are integrated by a software system and digitalized into real time images.

## b) ASPECT (ANNULAR SINGLE PHOTON EMISSION COMPUTERIZED TOMOGRAPHY

In principle, this technique is the same as SPECT, save that it utilises annular scintillating minicrystals having small diameters, so that such an apparatus can be limitedly used only for cranium tomography.

## c) PET (POSITRON EMISSION TOMOGRAPHY

In this case, particular nuclides ($C^{11}$, $N^{13}$, $O^{15}$, $F^{18}$) are purposely generated by nuclear accelerators (cyclotrons), by protonic bombardment of specific targets ($C^{13}$, $N^{15}$, $O^{16}$). Any generated nuclides are embodied in suitable molecules (water, sugars or amino acids) that concentrate or act in the body areas of interests.

Starting from the above indicated isotopes, radio pharmaceuticals are prepared and injected into the patients so as to accumulate in the carcinoma. The decay of a $\beta^+$ isotope typically generates a positron having an energy of $1 \div 2$ MeV that, at the end of its activity range (path) encounters an $e^-$ and annihilates, thereby emitting a pair of oppositely directed gamma radiations, with energy of 511 KeV.

The detection of the so generated gamma rays can be effected by means of a gamma-camera annular tomographic system, such as the above already described one, in "coincidence" in two diametrically opposite points and, therefore, it enables a collimator to be omitted, thereby increasing the sensitivity of the system.

Due to its complexity (short decay times of the isotopes: 2 to 110 minutes as half life time) and to its high cost (due to need that nuclear accelerators be installed near the hospital), such a system is exclusively employed in research centres, notwithstanding its good efficiency and the scarce risks for the patients.

All diagnostic techniques based upon radio pharmaceuticals were not suitable for application to the breast up to a few years ago, due to lack of suitable radio pharmaceuticals; only recently radio pharmaceuticals specifically useable for mammary investigations have been made available by pharmaceutical industries (such as beta emitting) $^{18}F$ fluorodeoxyglucose (FDG) and $^{99}Tc$ 2-methoxyisobutyl-isonitrile (99Tc MIBI) which emits a single gamma photon) and, therefore, the usual scintigraphic techniques ought now to be useable.

Unfortunately, however, due to technical reasons relating to the detection geometry and to the spatial resolution of the "gamma-cameras", it is certainly possible to form images of the tumor by means of such detection tool, but it is not possible to merge the scintigraphic images with the mammographic ones. In fact, when the presently available gamma-cameras are used, it is not possible to obtain the same projection of the mammography. Furthermore, the breast tumors at the T1 stage have a size of $0.5 \div 1.5$ cm; the spatial resolution of a commercially available gamma-camera is a about 1 cm, while the resolution of a mammograph is about 1 mm. Only when a high resolution gamma-camera is available, a scintigraphic image of a tumor of $0.5 \div 1$ mm size can be merged with a mammographic one.

It appears from the above explanations that it would be highly interesting to combine the very high spatial resolution of conventional systems, such a radiography and CAT (Computerised Axial Tomography), having a value up to 70-100 micrometers, with the power of a gamma-camera adapted to unequivocally evidencing the existence of a tumor (in a certain body function), so as to locate even the smallest metastases and to intervene at the first arising of the malignancy.

US-A-5 032 728 discloses a single photon emission computed tomography system comprising collimator modules arranged around the body to be imaged, a photo-detector assembly to detect the light radiation emitted from a scintillating crystal means as consequence of decay of certain isotopes and to generate signals proportional to the received light radiations and a hardware assembly to perform conversion and integration of all signals generated by said photo-detector system from an analog to a digital format as well as their amplification in order to subsequently couple them to an electronic processor which processes and displays them on a suitable monitor in the form of an image of the concerned body organ.

A drawback of this system is that, when it is used for tomography of body region including many organs (such as to rax) and some of these organs are more emitting than the organ to be examined, due to the fact that they absorb a greater amount of radio pharmaceuticals, then these more emitting organs generate a background radiation that can mask or even hide the radiation from the organ to be investigated.

The improvement principles as hereinbelow described are the ground of the subject-matter of this invention and it is an object thereof to realise a scintigraphic mammograph having a spatial resolution of about $200 \div 300$ micrometers.

Particular importance is given in this invention to using collimators: they affect the quality of the mammograph in so far as the detection efficiency and the discrimination of the starting position of the gamma radiation are concerned, so as to select only the radiation emitted from any tumoral cells existing in the breast that is being investigated.

Conventional gamma-cameras utilise lead or tungsten collimators characterised by parallel aperture or holes having a diameter greater than or equal to 1.3 mm and separator baffles having a thickness > 200 micrometers. Use of such collimators together with a newly proposed detector would cause distortion of the generated images, due to the sub-millimetric spatial resolution.

The improvement as proposed in the field of collimators relates to use of a high atomic number (Z) material, with aperture or holes having a diameter in the range between 0.5 and 1 mm and with separator baffles having a thickness in the range of about $0.1 \div 0.2$ mm and with a thickness matched to the energy contents of the incident gamma radiations.

The use of a new generation of oblique collimators is additionally suggested as an aspect of this invention aimed at reaching the following results:

    a) oblique collimators together with the multi-plane construction of the detector, as hereinbelow described, allow to track the path followed by the X-rays, thereby enhancing the capability to establish the position of the tumoral cells,

    b) collimators having oblique apertures or holes alternatively extending in two different directions enable a double tracking to be achieved, so as to permit to measure the third co-ordinate of the X-ray source, that is the depth of the tumor,

    c) collimators consisting of a combination of parts, each comprising oblique apertures or holes, enable to project the image in the form of different spaced apart images, so as to as to overcome the limitation caused by the so called dead areas of the photo-detectors. Said dead areas, in fact, are an obstacle to be solved in making gamma-cameras of noticeable sizes.

Particular importance is also given in this invention to use of scintillating crystals and fluorescent or optical fibers.

Conventional gamma-cameras utilise a single planar crystal, on the surface of which a number of photo-multiplier are adhered. The accuracy of the information concerning the arrival position of the particle is jeopardised by the crystal thickness, within which the light is subject to spreading effects, as well as by any dead areas existing between a photo-multiplier and the adjacent ones.

In a first embodiment, according to this invention, it is suggested to use scintillating crystal of a novel type, having such chemical and physical properties as to enable minicrystals of sizes up to 0.3 x 0.3 sq. mm in cross-section, to be used, arranged in multi-crystal arrays having a thickness of about 10 mm, with separator baffles between crystals having a thickness of about 10 micrometers and a sufficient efficiency as to detect gamma radiations with energy $\geq$ 30 KeV.

In other embodiments, X-ray and gamma radiation responsive constructions are provided, wherein the light information is not spread or dispersed and no dead area exists. Such constructions can be made as any one of the following types:

1) overposed planes alternatively consisting of planar scintillating crystals and plastic fluorescent fibers, with square cross-section shape, arranged in orthogonal directions, a fiber plane with respect to the adjacent ones.

A sandwich structure is obtained wherein the basic element (X-directed fiber - scintillating crystal - Y-directed fiber) can be repeated a number of times so as to reach the desired efficiency. The base of the structure has dimensions of about 20 x 20 sq. cm. The thickness of the planar crystals is of about $0.2 \div 2$ mm and the dimensions of said fluorescent fibers of $0.5 \div 3$ mm side enable a punctual sampling of the scintillator light to be effected, in view of the fact that no dispersion of the light occurs.

The concerned planar crystal can also be used in the form of arrays of discrete minicrystal having dimensions in the range from 0.3 x 0.3 mm and 2 x 2 mm, a thickness in the range between 0.2 and 10 mm, with separator baffles for optical separation between the crystals in order to avoid cross-talk interferences, having a thickness in the range from 10 micrometers and 200 micrometers.

Since the fibers completely cover the surface of the scintillators and convey the luminous information along an horizontal photo-multiplier plane arranged at the side of the sandwich structure, no dead zone exists.

Further optional photo-multipliers arranged under the multiplane structure collect any light not conveyed within the fibers.

2) Planes arranged side-by-side in vertical or oblique direction, rather than in horizontal direction, comprising planar scintillating crystals and possibly plastic fluorescent fibers, having a square cross-section shape and arranged in parallel direction, a fiber plane with respect to the adjacent ones. In this case, the sizes of each crystal are of about 0.5 mm x 10 mm x 200 mm. The crystals and any fiber convey the luminous signal in vertical direction toward a photo-multiplier or CCD system and the crystals also transmit light in horizontal direction to photo-multipliers positioned at their sides.

With vertically extending crystals and fibers, it will be necessary to move the gamma-camera and to sequentially run two or more collection sequences according to whether the cross-section area of the fibers is equal or greater than the area of the scintillating crystals. With sloped crystals and fibers, it is not necessary to move the gamma-camera for a total collection to be achieved, since there are no dead areas caused by the fibers because, upon giving the sloping angle, the concerned ray will hit at least one scintillating crystal. These approaches enable the signals conveyed by the fibers to be read both by means of photo-multiplier and by means of CCD (Charge Coupled Devices) systems.

3) Overposed layers alternately comprising planar scintillating crystals and lens or optical fiber matrixes, arranged in orthogonal direction with respect to the crystal plane. Said optical fibers should have such a numeric aperture as to permit the light to pass according to predetermined angles. The possibility is thereby realised to restrict the output cone for the light emitted by the crystal, which results into an accurate spatial information. When lenses are used (or fibers with controlled refraction index) the concerned light is conveyed in a direction orthogonal to the crystal, without enlargement of the light cone. The light is collected in vertical and horizontal directions by photo-multipliers.

4) Overposed planes of scintillating crystals and multiple layers of materials having such a refraction index as to determine a certain maximum angle for total reflection and to transmit the crystal light only within fixed angle cones. Also in this arrangement, the light is collected in vertical and horizontal directions by means of photo-multipliers.

5) Microfibers of scintillating crystal arranged side-by-side so as to form a matrix wherein the luminous information is concentrated in a single microfiber and it is conveyed to the photo-multiplier directly and/or by means of optical or fluorescent fibers.

Aiming it solving the problem of the dead areas, caused by inactive regions existing between side-by-side arranged photo-multipliers, scintillating crystals having different response speed characteristics are laterally combined with the single crystal matrix, in order to exploit the so-called Phoswich method. In a further implementation, fibers to convey the light in an horizontal direction can be combined with such single crystal matrix to the same effect.

In all approaches such as the previously described ones, or any combination thereof adapted to realise a high spatial resolution detector, achieved by means of a controlled dispersion of the luminous information, the issue of the dead areas generated by any inactive regions of side-by-side arranged photo-multipliers is overcome by means of any one of the following approaches:

a) oblique collimators;
b) oblique scintillating crystals;
c) optical and/or fluorescent fibers having divergent structures or variable cross-section sizes, or fibers simply conveying the signal corresponding to scintillating crystal overposed to dead areas of the concerned photomultiplier toward other photomultipliers.

Particular importance is also given in this invention to using a position sensitive photo-multiplier system, which comprises an anode set or generally light sensitive elements, of rectangular, circular or square shape, independent on one another or consisting of crossed wires.

In the present invention, it is suggested to use a position sensitive photo-multiplier (PSPMT) with a crossed wire anode at a pitch of 3.75 mm or less, so as to be responsive to signals emitted by single fibers involving a single pixel, thereby enabling the spatial resolution to be increased; in a different approach, it is suggested to use a matrix or array of solid state detectors, such as silicon or germanium photo-diodes with elements or pixels in the range between 0.2 x 0.2 mm and 1 x 1 mm, preferably between 0.5 x 0.5 mm, adapted to read photons, in order to discriminate the detected signal and, therefore, to determine the location and to detect the energy absorbed by the scintillating crystal in its interaction with the gamma ray, at a net resolution up to about 0.3 mm.

According to this invention, the light generated by scintillation is conveyed to each anode or to each portion of the photo-multiplier system directly from the crystal or by means of light guides or one or more optical or fluorescent fibers, so that the position and the energy released while the particle is passing through the crystal are determined by the method based upon a computation of the charge centroid and by the sum of the light amounts emitted by each fiber, respectively.

The best spatial resolution achievable is about 0.3 mm. Furthermore, this approach permits to avoid any dead areas caused by the containment walls of the single photo-multiplier, as well as to utilise a restricted number of photo-multipliers, thereby decreasing the active cross-section area by means of light guides and optical fibers.

According to this invention, the combined use of an electronic system to integrate all of the generated signals and of a suitable software allows to achieve real time images of the investigated body organ.

In conclusion, the scintigraphic mammograph according to this invention enables the modem scintigraphic techniques to be applied to investigating the breast carcinoma by utilising suitable radio pharmaceuticals (technetium based or other developable types) capable to be preferentially bound to tumoral cells and emitting low energy (about 140 KeV) gamma radiations with useful even if short (6 hours) half life times. This is made possible because the system according to this invention combines a high conversion efficiency of the gamma rays, as it is achieved by using minicrystals or a number of scintillating planar crystals with a high spatial resolution. This high spatial resolution is achieved as a result of various factors, namely the use of multicrystal matrixes or arrays of extremely small sizes, or the small thickness of the crystals, within which no dispersion of the light can take place, thereby maintaining a punctual information upon the transition of the particle; the combination of the crystals with fluorescent fibers, which store the scintillation position within the crystal; as well as the employment of position sensitive photo-multipliers, by which the single fluorescent fibers conveying the luminous signal are detected.

This system enables to evaluate without the smallest doubt whether tumoral cells exist in the investigated breast and the evaluation is made with such a spatial resolution (< 1 mm) as to locate even the smallest metastases and, therefore, the very first appearance of the malignancy.

The high efficiency and spatial resolution of the system according to this invention permit to use technetium based radio pharmaceuticals capable to emit low energy (140 KeV) gamma radiations having a short half like time (6 hours) and, as such, scarcely dangerous for the patients; furthermore, they enable the scintigraphic image of the investigated tumor (having a size of 0.5 ÷ 2 mm) to be "merged" with the mammographic (radiographic) image, thereby reaching an absolute of diagnostic certainly.

Specific subject-matter of this invention is an apparatus for carrying out scintigraphic mammography with submillimetric spatial resolution by detecting gamma radiations with 30 KeV to 2 MeV energy, comprising, in a sensitive head section (10) with a box shaped shielding container (16):

- an apertured collimator (11) for collimating the gamma radiation emissions from the body organ to be investigated;
- a scintillating crystal structure (12) for converting the gamma radiations emitted from the concerned body organ into light radiations;
- a photo-detector assembly (13) to detect the light radiations emitted from said scintillating crystals and to generate signals proportional to the received light radiations and
- a hardware assembly (15) to perform conversion and integration of all signals generated by said photo-detector assembly from an analog to a digital format as well as their amplification in order to subsequently couple them to an electronic processor which processes and displays them on a suitable monitor in the form of an image of the concerned organ,

characterized in that:

- said collimator (11) is of a high atomic number material selected among lead, tungsten and like and it has apertures having a diameter in the range between 0.5 and 1.5 mm, particularly 0.6 mm, as well as aperture separation baffles with a thickness in the range between 0.05 and 0.5 mm, particularly 0.1 mm;
- said scintillating crystal structure (12) comprises a matrix of minicrystals made of material selected among YAP : Ce, Nal, Csl, BGO and like, particularly YAP : Ce, they have a cross-section dimension in the range between 0.3 x 0.3 and 2 x 2 mm, particularly 1 x 1 mm, with a thickness in the range between 0.5 and 20 mm, particularly 10 mm; the individual minicrystal of said matrix are optically separated from one another and any dead area between the individual minicrystals is in the range between 2 and 0.5 μm;
- said photo-detectors (13) are position-sensitive photo-multipliers having a crossed wire anode with a pitch of 3.75 mm or less or position-sensitive solid state detectors,
- a pressure member (17) is provided to compress the body organ to be investigated against the collimator (11) arranged at the lower face of the head section (10).

In a preferred embodiment, said scintillating crystal structure comprises a matrix of minicrystals and a bundle of optical fiber light guides is provided to optically couple said scintillating minicrystals to said photo-multiplier assembly.

In a further preferred embodiment, said scintillating crystal structure comprises a layered plane structure including planar scintillating crystals and fluorescent fibers and the scintillation light is read in orthogonal and parallel directions with respect to the crystals by means of photo-multiplier devices adapted to receive and read both the light emitted from the crystals and the light emitted from the fibers.

It is also specific subject matter of this invention a system for scintigraphic mammography with sub-millimetric resolution, comprising

- means to collimate the gamma radiation emitted from the body organ to be investigated;
- scintillating crystal means to convert said gamma radiations into ultraviolet-visible light;
- means to convey said ultraviolet-visible light from said scintillating crystals to photo-multiplier means;
- photo-multiplier or solid state detector means to convert said ultraviolet-visible light into analog electric signals;
- means to pre-amplify said analog signals;
- means to power supply said photo-multiplier means, to amplify the acquired signals and to convert said signals from analog to digital format; and
- means to process said digital signals and to form the image based thereupon.

Further particulars and advantages as well as characteristics and construction details will be evident from the following description with reference to enclosed drawings wherein the preferred embodiments are shown by way of illustration and not by way of limitation.

In the drawings:

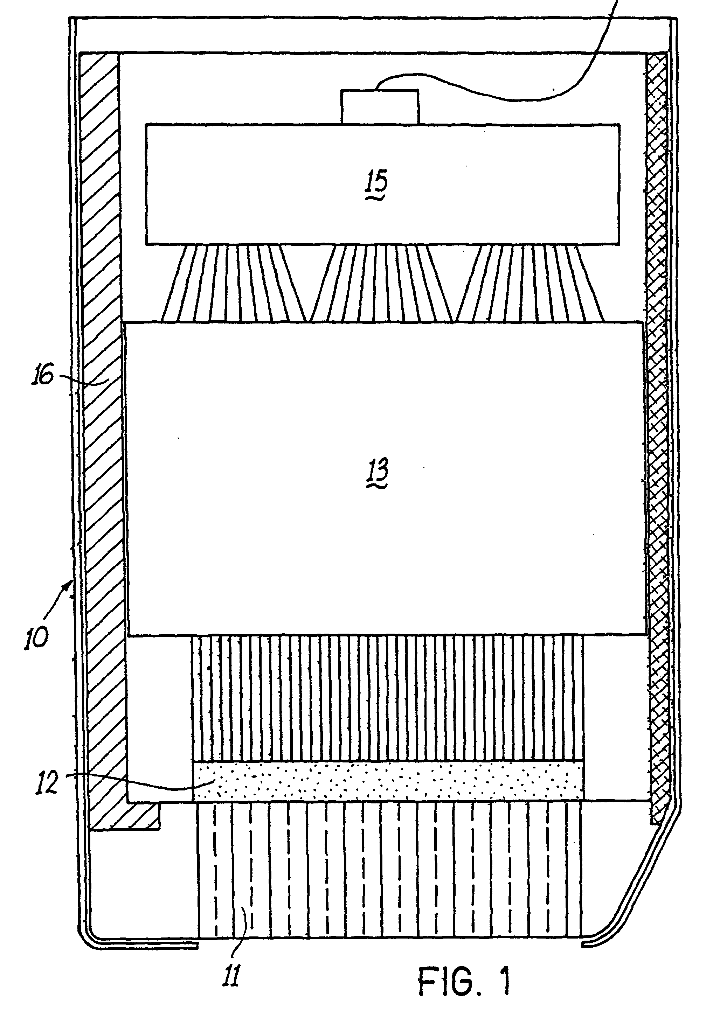

Figure 1 is a cross-section view of the head portion of a mammograph according to this invention;

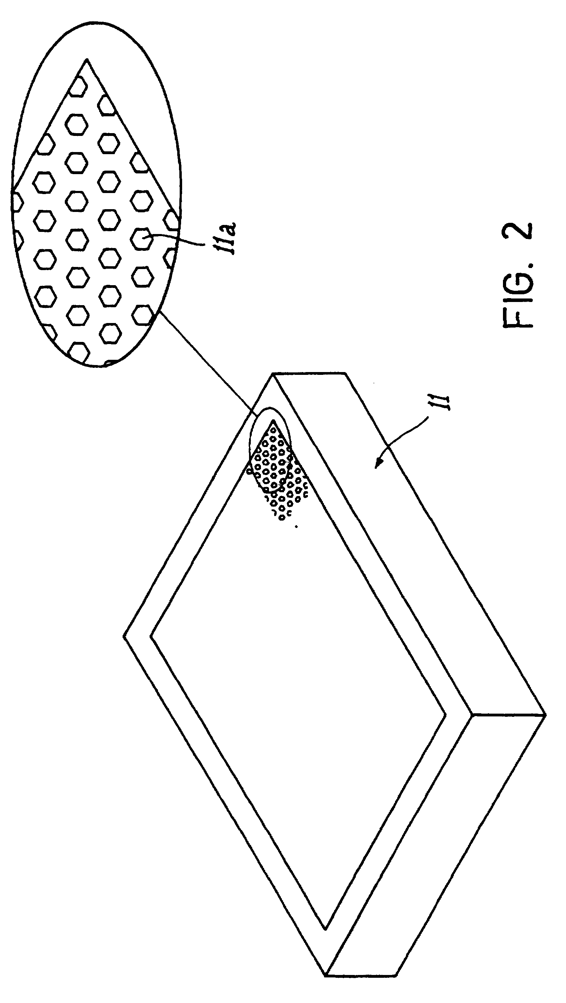

Figure 2 is a perspective view of the collimator, with an enlarged detail of the apertures or passage holes;

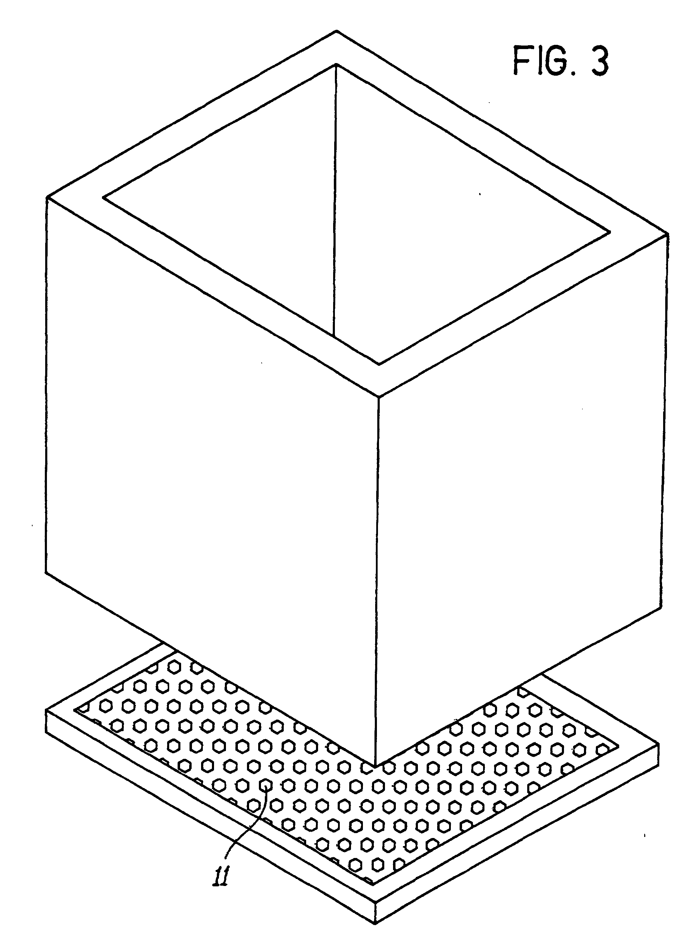

Figure 3 is a perspective exploded view of the assembly comprising the collimator and the housing box or shield;

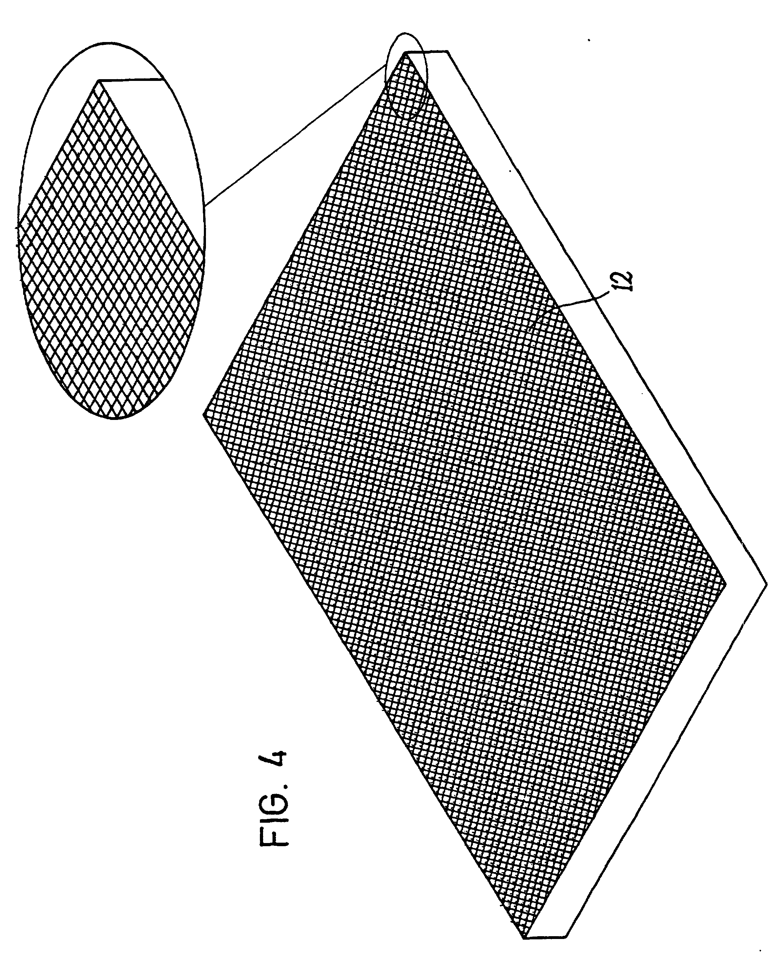

Figure 4 is a perspective view of the scintillating minicrystal array, with an enlarged detail;

Figure 5 is a view of the optical fiber bundle groups for connecting the photo-multipliers and the pre-amplifier;

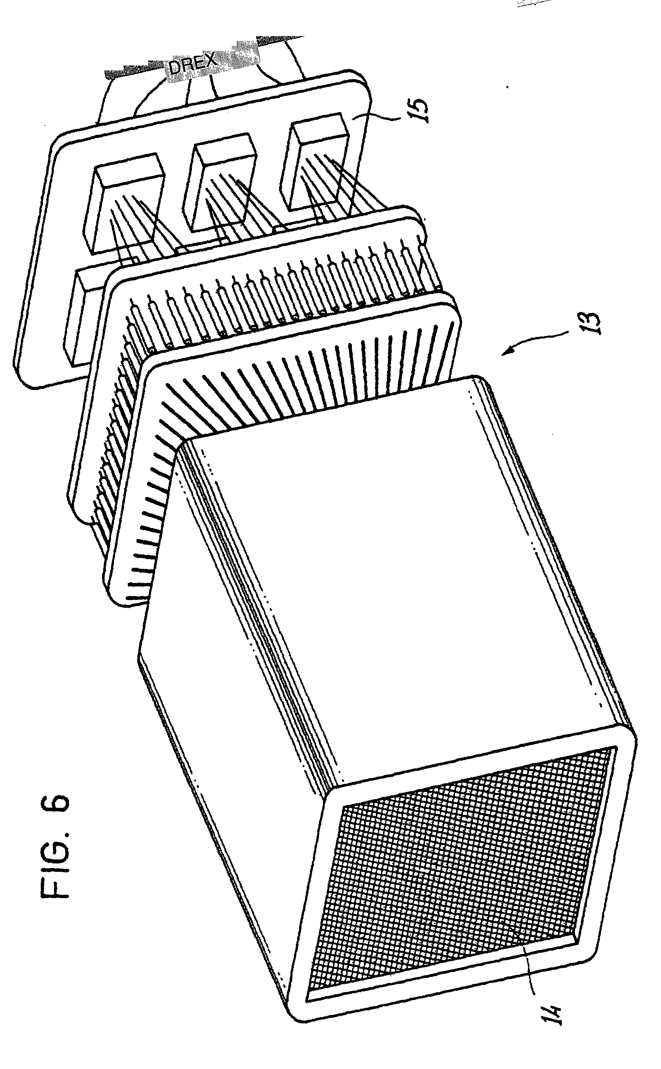

Figure 6 is a perspective view of the photo-multiplier with related pre-amplifier;

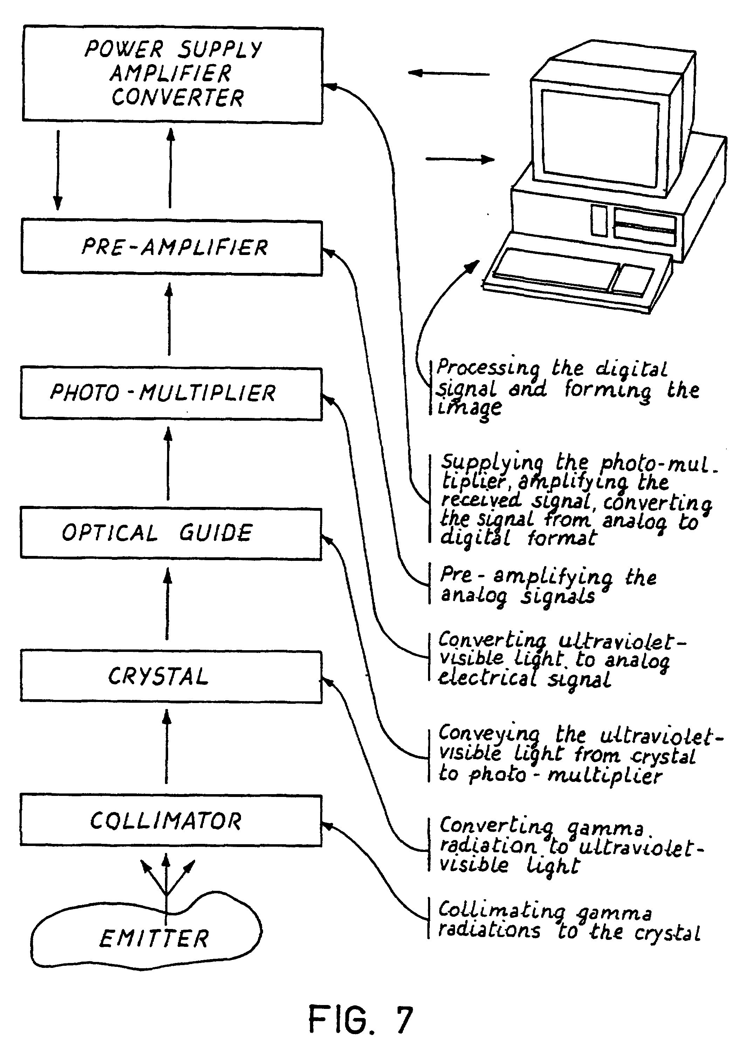

Figure 7 shows a block diagram of the head portion of a mammograph according to this invention in its cooperation relationship with a computer to process the digital signal and to form the image;

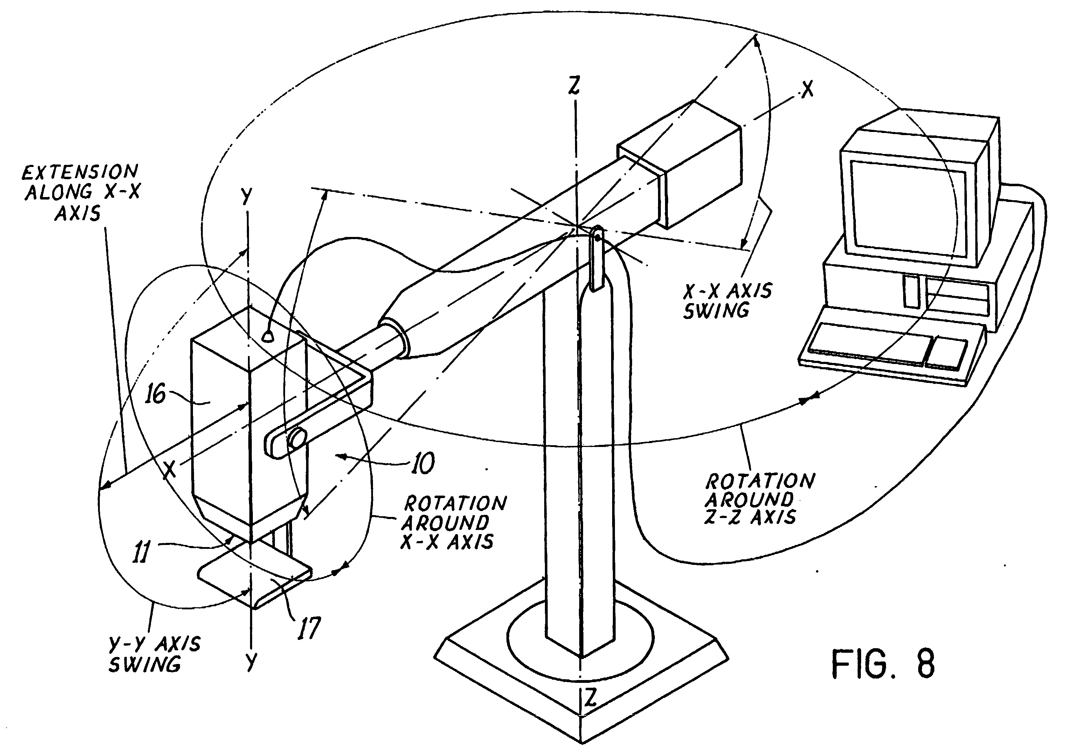

Figure 8 is a perspective view of the assembly of the mammograph with the digital processor co-operating therewith and with the support and swinging mechanical structure;

Figure 9 shows a block diagram of the circuit for sampling and processing the output signals of the position sensitive photo-multiplier;

Figure 10 shows a block diagram of a second embodiment based upon employment of resistor chains;

Figure 11 shows a block diagram of the circuit comprising a threshold read system;

Figure 12 is a schematic view of the coupling geometry between minicrystals and optical fibers;

Figures 13A and 13B are cross-section views taken from two orthogonal directions of the head portion of the mammograph according to a different embodiment of this invention;

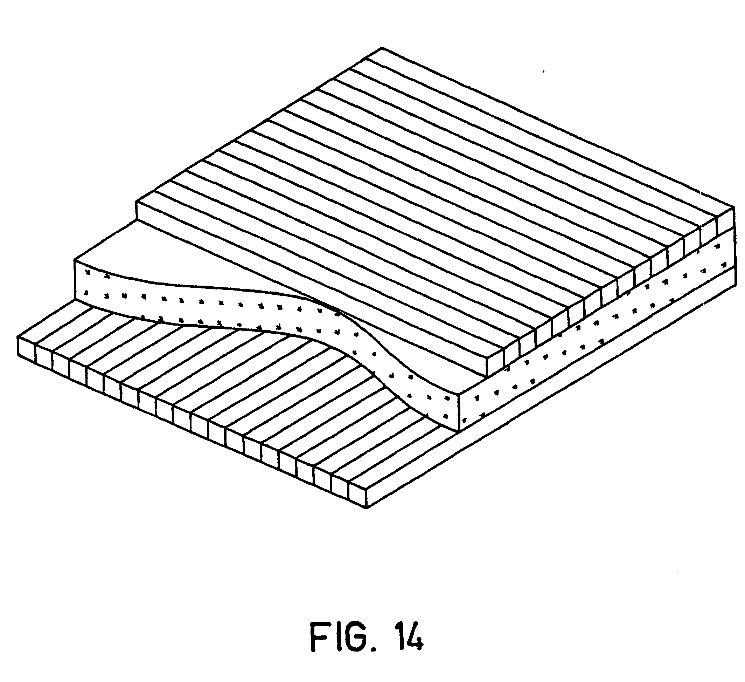

Figure 14 is a perspective view of the sandwich assembly of scintillating crystals and fluorescent fiber planes;

Figure 15 is a view of the connection system between the multi-anode photo-multiplier and the various fluorescent fibers.

By referring now to the drawings, the mammograph according to this invention comprises, as one of its essential component parts, a sensitive head 10 wherein the component parts to be subsequently described are housed.

A collimator 11 made of a high atomic number material (typically Pb, W and like) with cross-section dimensions of about 250 x 150 mm (corresponding to the dimensions of the radiographic cassette of conventionally used mammographs) or like and with a thickness in the range between 10 mm and 40 mm, preferably about 20 mm, is shown. Collimator 11 has holes 11a with diameters in the range between 0.5 and 1.2 mm, preferably about 0.6 mm, a separator baffle being provided between said holes having a thickness in the range between 0.05 mm and 0.3 mm, preferably about 0.1 mm. Said collimator is designed to singularize the gamma radiation emissions from the concerned body organ and acts as a strict spatial selector.

It is not strictly necessary for the apertures or holes of the collimator to be parallel. In fact, they can be made according to a convergent, divergent or localising structure or as a pin-hole pattern.

An array 12 of X-ray responsive, scintillating mini-crystals is arranged upon said collimator 11. This array or multi-crystal matrix 12 has dimensions such as to correspond to the dimensions of the collimator and particularly, for instance, 250 x 250 mm or less, with single YAP : Ce mini-crystals having dimensions in the range of 2 x 2 mm up to 0.3 x 0.3 mm, in particular 1 x 1 mm, with a thickness in the range of 0.5 mm and 20 mm, in particular 10 mm, optically separated from one another by means of light opaque and reflecting baffles having a thickness in the range between 2 micrometers and 0.5 mm, in particular about 10 $\mu$m, adapted to convert gamma rays of 2 MeV to about 30 KeV energy into light of such a wave length as to be suitable to be read, in the range between about 350 nm and about 600 nm.

The concerned scintillating crystals may be made of Nal, Csl, BGO and like.

A bundle of light guides or optical fibers is associated with said array of scintillating mini-crystals, and the related light guides or optical fibers are made of plastic material, such as PMMA, PS, PC and like, or of non-plastic material, such as glass, silica, quartz and like. The bundle has a cross-section area of 250 x 150 mm or less and comprises single fibers of hexagonal or square cross-section shape, with side dimensions of about 1 mm and anyway in the range between 0.5 and 2 mm. The fibers are optically separated from one another by means of a reflecting coating deposited upon their interface, so as not to leave voids within the bundle and not to enable the light conveyed in a fiber to interfere with any contiguous fiber.

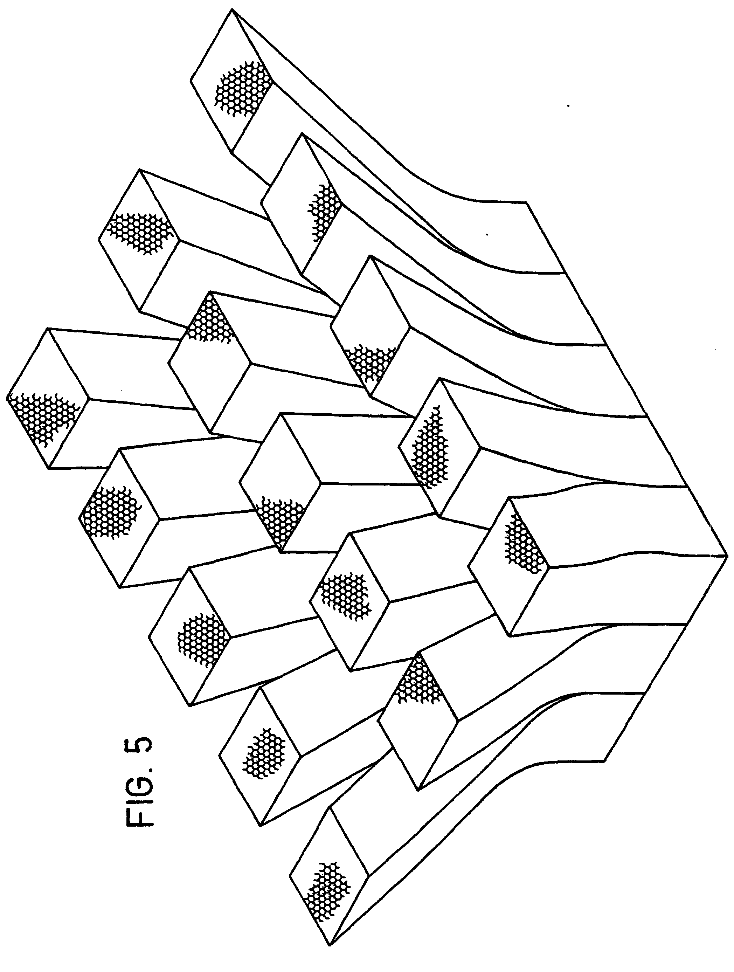

The base of the optical fiber bundle is optically coupled to the scintillating crystal matrix 12, while, at its opposite end, in a materially implemented embodiment, as shown in Figure 5, it is divided into fifteen sub-bundles, each having a square cross-section shape of about 50 x 50 mm, optically coupled to as many position sensitive photo-multipliers 13, with a useful window of about 50 x 50 mm size (in the case of a scintillating crystal matrix of 180 x 180 mm area, the bundle could be divided into nine sub-bundles, each having a square cross-section shape of about 60 x 60 mm, optically coupled to as many position sensitive photo-multipliers, with active window of 60 x 60 mm); in the case of a scintillating crystal matrix of 180 x 120 mm size, it could be divided into six sub-bundles, each having a square cross-section shape of 60 x 60 mm, optically coupled to as many photo-multipliers.

Summarising, the optical fiber bundle utilised in this invention has at its base a cross-section area corresponding to the net surface of multi-crystal matrix and, at its opposite end, it is divided into a number of sub-bundles, each having a cross-section similar, both in shape and in dimensions, to the active window of a plurality of photo-multipliers or solid state detectors and a cumulative cross-section area equal to its base cross-section area or also a smaller cross-section area adapted to be coupled to a single photo-multiplier or solid state detector and a frusto-conical or frusto-pyramidal shape. In this case, the geometric shape of the base, whether square or rectangular, hexagonal, cylindrical and like, is not necessarily maintained at its top.

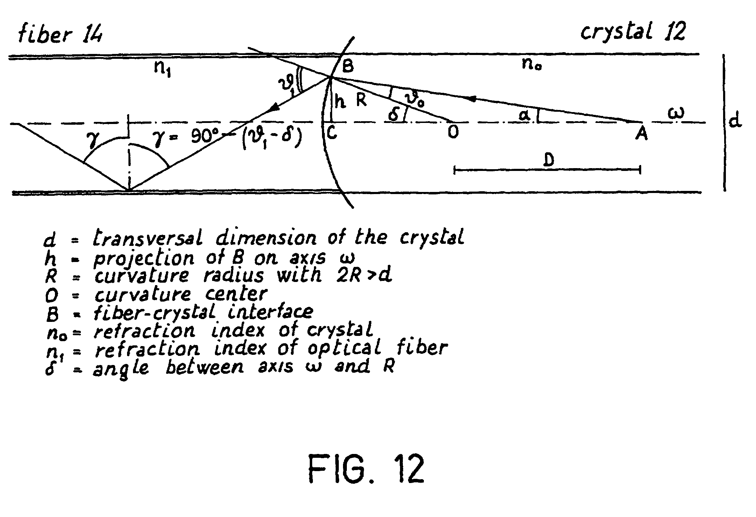

It should be noted that the coupling interface between the scintillating mini-crystals and the optical fiber bundle should be such that the light coming from said mini-crystals while passing within the fibers do not be subjected to deflections due to refraction index changes, which cause significant decreases in the overall efficiency of the system. The concerned scintillating mini-crystals, therefore, are suitably individually machined according to their dimensions and refraction index and the individual fibers are then perfectly coupled to their geometry as described in Figure 12 in respect of a crystal and a fiber having a spherical curvature at their interface.

The light ray originating from point A, which is located at a distance D from the curvature centre O, forming an angle $\vartheta_o$ with a line normal to interface B and forming an angle $\alpha$ with axis $\omega$ of the crystal (12), will be deflected at it passage through the optical fiber (14) and it will form an angle $\vartheta_1$ with respect to a line R normal to the interface point B, such that the angle $\gamma$ formed by the reflected light ray with a line normal to the outer surface of the optical fiber is greater than the angle limit, in order that the light can be conveyed along minimum length paths.

Identical considerations apply for preferred paraboloidal, ellipsoidal or pyramidal profiles of the crystal head sections.

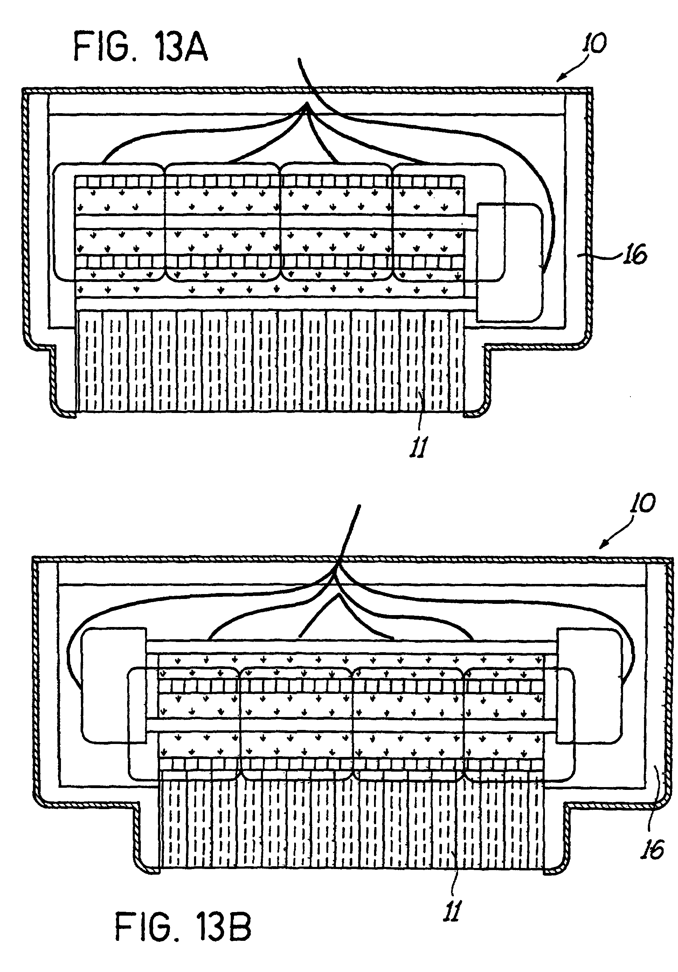

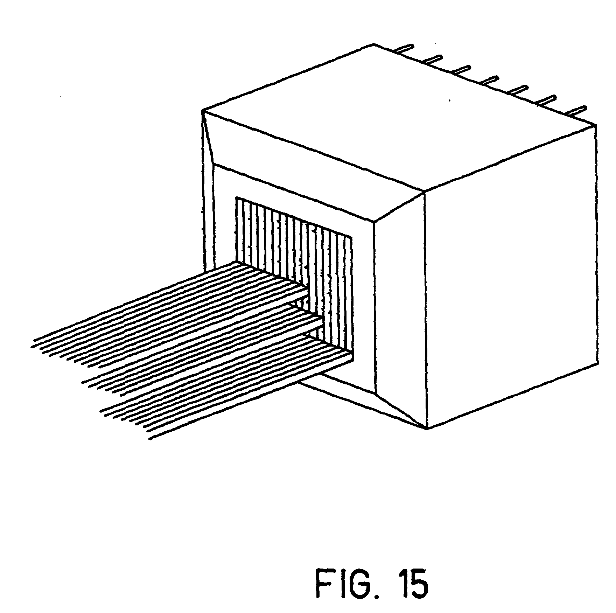

In the embodiments shown in Figures 13A, 13B and 15, a sandwich structure 12 comprising 12 gamma ray responsive scintillating crystals and fluorescent fibers is arranged upon collimator 11. Structure 12 has dimensions corresponding to the dimensions of said collimator and particularly, for instance, 250 x 150 mm or like, with a crystal thickness in the range between 0.2 mm and 2 mm, preferably 0.5 mm, and a surface area in the range between 50 x 50 sq. mm and 400 x 400 sq. mm, preferably 200 x 200 sq. mm, adapted to convert gamma rays of 2 MeV and up to about 30 KeV energy into light of such a wavelength as to be amenable to be collected and conveyed by said fibers, in the range between 200 nm and about 570 nm. The crystals by which said structure is formed are made of a material selected among YAP: Ce, Nal, BGO and like, preferably Nal (TI).

Two planes or layers of fluorescent fibers of square cross-section shape are coupled to each crystal. The fibers of a plane are arranged in an orthogonal direction with respect to the fibers of the other plane or they form a different angle so as to obtain two-dimensional information. By means of an accurate investigation, it is realised that it is possible to achieve a two-dimensional image of the object to be analysed together with an evaluation of its depth or distance from the detector by means of a multi-plane or multi-layer structure, based upon three possibilities: a) on each plane, images are obtained, consisting of the intersections of planes with a cone having the object being analysed at its apex; b) in view of the particular collimators used therein, particularly crossed or localising collimators, the image is localised upon one of the planes of the multi-plane structure; c) tomographic images are obtained by means of curved surfaces, particularly paraboloidal, cylindrical or anatomic surface, as it is made possible by the mechanical characteristics of the plastic fibers.

Crystals can be coupled with a binder or in air without a binder. The surface of the crystals can be, but it is not necessary for it to be covered by a multi-layer consisting of a material having such a refraction index as to create an anti-reflection surface and to optimise the percentage of the crystal scintillation light that hits the fibers, also taking the angle between the light ray and the surface of the crystal itself into account.

The lateral surface of the fibers, which are perpendicular to the surfaces of the crystals, can have, but it is not necessary for them to have a cover layer of reflecting material, so as to avoid any passage of light from a fiber to the adjacent ones.

At both ends, the surface of the fibers is machined so as to obtain an "optical" finish.

The fibers are coupled at both ends to multi-anode photo-multipliers.

Alternatively, one of the end surface is covered by a high reflectivity material and only the other end surface is coupled to the photo-multiplier.

In stead of a planar crystal, it is possible to utilise a number of scintillating side-by-side arranged tiles, having square cross-section shapes (of dimensions in the range between 0.3 mm x 0.3 mm and 50 mm x 50 mm) or rectangular shapes with minor side dimensions in the range between 1 mm and 40 mm and major side dimensions up to 250 mm, or any other shape. The tiles can be optically separated, the dead distance between single crystals being in the range between 2 μm and 0.5 mm, particularly 10 μm; the thickness of these tiles being in the range between 0.2 mm and 10 mm, particularly 0.5 mm.

Optical tiles of such kind, of plastic or non plastic materials, are coupled to the lateral surfaces of the scintillating crystals and convey any light collected by them to multi-anode photo-multipliers, thereby acting as light guides, possibly having their section facing to the photo-multiplier smaller than the one facing to the crystals, so as to reduce their volume and to more easily identify the fluorescent fibers coupled to the scintillating area.

The mask used to guide the fibers to the photo-multiplier can be made in compliance with such a logic as to minimise the active area of the multi-anode photo-multipliers used. This means that a number of fibers can be associated to a single anode and any discrimination of the fibers conveying luminous signals is based upon use of information furnished by optical tiles or upon the periodicity of the anodes that are hit by a luminous signal (such a periodicity derives from the periodicity with which the fibers are associated to photo-multipliers).

The concerned fibers are made of polystyrene, PC, PMMA, glass or silica, quartz or like.

The above mentioned planar crystals can be arranged as a matrix or an array of single mini-crystals having dimensions in the range between 0.3 x 0.3 mm and 2 x 2 mm, a thickness in the range between 0.2 mm and 10 mm, with separator baffles to optically separate crystals by adjacent ones, in order to avoid any cross-talk effect, and a thickness in the range between 10 μm and 100 μm.

Since the fibers completely cover the surfaces of the scintillators and convey the luminous information along an horizontal plane or layer to photo-multipliers positioned aside the abovesaid sandwich structure, no dead area exists any more.

Possible photo-multipliers arranged under the multi-plane or multi-layer structure collect any light not conveyed within the fibers.

The above described implementation is not the sole possible implementation, but further embodiments, for instance the ones already mentioned in the introductory portion of this specification, are possible according to the applications, according to the dimensions of the detector, to the energy in the particles to be detected, to the spatial resolution to be achieved, as well as to the costs to be incurred.

As above mentioned, important component parts of this invention are the position sensitive photo-multipliers: they are *per se* known devices, that are defined as "position sensitive" in view of the fact that their "head" is formed by unique structures, both in respect of their multiplication section and in their charge collecting section. The multiplication section comprises overposed planes or layers of fine and closely spaced apart mesh electrodes or of regularly apertured foil electrodes. These structures with aligned apertures or holes are such that the electrons follow rectilinear paths, characterised by a reduced lateral dispersion: under such assumption, the transit time of the electrons from the photo-cathode to the anode is noticeably reduced (in the range of nanoseconds or of tens of nanoseconds), the paths are almost insensitive with respect to external magnetic fields and additionally the electron clouds formed by photo electrons emitted in various positions from the photo-cathode are spatially distinct. After the multiplication stage, the charge is sampled by means of a structure realised by means of a crossed wire network or, as in the hereinbefore described application, by means of a square, rectangular or circular element assembly, each of which is optically separated in respect of the other ones, so as to form a single reading channel.

Alternatively, photo-multipliers having not only discrete anodes, but also discrete windows, cathodes and dynodes can be used.

In advantageous embodiments, the solid state photo-multiplier or detector assembly can be comprised of one or more single solid state photo-multipliers or detectors and each photo-multiplier can comprise many anodes or one crossed wire anode with a pitch of 3.75 mm or less. Furthermore, the solid state detectors can be made of silicon photo-diodes or other material or of CCD cameras with pixel dimensions in the range between 12 x 12 micrometers and 2.54 x 2.54 mm adapted to read light photons in order to determine the position and the energy released in the scintillating crystal.

In the embodiment comprising fifteen photo-multipliers, as it is shown in Figure 5, they have an active window of about 50 x 50 mm size and they are optically coupled to fifteen sub-bundles of optical fibers having a cross-section area of 50 x 50 mm. In the embodiment comprising nine or six photo-multipliers, they have a crossed wire anode (14) with a pitch of 3.75 mm and an active window of 60 x 60 mm and they are optically coupled to nine and to six sub-bundles of optical fibers, respectively, with a square cross section area of about 60 x 60 mm size.

Further hardware components of the structure of the mammograph according to this invention are made by components generally known to those skilled in the art and namely a pre-amplification section 15 for the analog signals and a section having the function to supply the photo-multiplier, to amplify the detected signals and to convert the analog signal to digital format, as it is required for processing in computers for definitively form the image to be displayed.

Furthermore, an amplification of the crystal scintillation light can be performed by exploiting the stimulated emission phenomenon, realised by means of a pumping action exerted by a laser or a lamp.

The operation of the processor and then the operation of the whole electronic hardware system for reading and integrating the analog signals generated by the photo-multiplier dynodes, for subsequent conversion to digital signals, is governed by a suitable software which definitively provides for processing the concerned digital signals and for displaying the images in real time on a suitable monitor having a spatial digitalisation resolution of 0.5 mm and up to 0.1 mm.

All mechanical, optical and electronic components of the hereinabove described mammograph are housed in a suitable box shaped shield 16 that in effect is to be considered as the external container of the head section 10 and that prevents the gamma rays from reaching the scintillating crystals or the photo-multiplier from positions other than the ones directly facing the collimator.

As it usually occurs, the head section 10 of the mammograph is combined with a pressure member 17 acting to compress the breast to be investigated against its lower sensitive face (collimator). The mechanical section further comprises a support structure that enables the sensitive head section and the pressure member associated and rigidly connected thereto to move along three spatial co-ordinates, so as to effect mammographic analysis not only in vertical, but also in oblique and lateral directions.

The same principles on which the present invention is grounded can also be extended to other applications, such as the evaluation of the biologic functionality and the tomography.

As far as the evaluation of the biologic functionality is concerned, this invention can also be exploited in order to carry out functionality tests of a certain anatomic organ, for instance to establish the clinical death of a patient, some organs of which are to be extracted for subsequent transplant. In the latter case, cerebral functionality tests will be carried out.

As far as tomography is concerned, the sensitive head section according to this invention can be mounted upon a mechanical structure such that also a rotation of 360° is made possible and it can also have a ring shape with suitable diameter and width values to also effect a SPECT type tomography.

In all above mentioned applications, the apparatuses will be provided with electronic circuitry adapted to integrate all signal generated by photo-multiplier dynodes as well as with a software adapted to display the digitalized signal as images in real time, with a spatial resolution up to 0.2 mm.

Before discussing the details of the electronic circuitry according to this invention, it will be useful to give some explanations about position sensitive photo-multiplier tubes.

Such tubes have all general characteristics of conventional photo-multiplier tubes. The characteristics distinguishing the tubes as used in this invention with respect to conventional photo-multiplier tubes enable them to locate the positions of luminous photons hitting the photo-cathode area. The charge multiplication stage is made so as to enable rectilinear paths to be followed by the electrons during multiplication. In this circumstance, the transit time of electrons from photo-cathode to anode is noticeably decreased (about a few nanoseconds or tens of nanoseconds), the paths are almost unresponsive to external magnetic fields and, additionally, the electron clouds formed by photo-electrons emitted in different position from the photo-cathode are spatially distinct. After the multiplication stage, the charge is sampled by a multi-anode structure or by a crossed wire anode.

The electronic hardware designed for reading and firstly processing the charge signal collected upon the various anodes or upon the anode wires of the photo-multiplier can be implemented in two different ways.

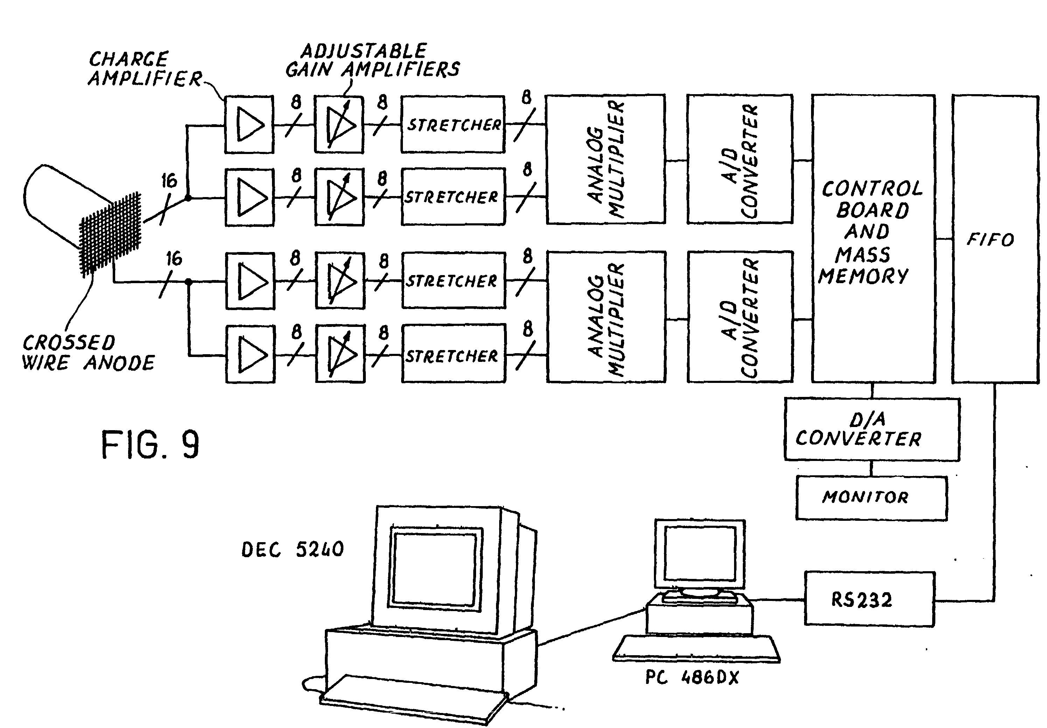

In a first embodiment, a system is provided for sampling and processing the signal in association with each anode or each wire. In Figure 9, a block diagram is shown to illustrate the electronic circuit system designed for sampling the output signals of the position sensitive photo-multiplier. The charge collected by the anodes or by the anode wires is integrated and amplified by a double stage of pre-amplification and variable gain amplification. The characteristic time for integrating the charge, in this embodiment, is about 10 milliseconds: this makes it possible to completely collect the charge and establishes the maximum acquisition rate at 100 Hz, in order to avoid signal overlapping effects. The outputs of the amplifiers are coupled to stretcher circuits, which further extend the pulse signals to be subsequently transmitted to an analog multiplexer circuit.

Two multiplexer circuits are provided in this apparatus and each of them processes the signals coming from an anode or a wire assembly of the photo-multiplier in respect of the two co-ordinates. Each multiplexer circuit sends its output signals to a 10 bit analog-digital converter. At this point, the digital data are stored in a mass memory system and are processed by the master processor which organises them into strings, each associated with an interaction event in the crystal. The strings are then shifted in FIFO memory registers and are then transmitted through a serial interface RS232 to a Personal Computer and to a computer station, by means of which the necessary processing operations can be carried out.

Digital data, upon conversion into analog format, can be extracted from the mass memory and displayed upon a suitable monitor.

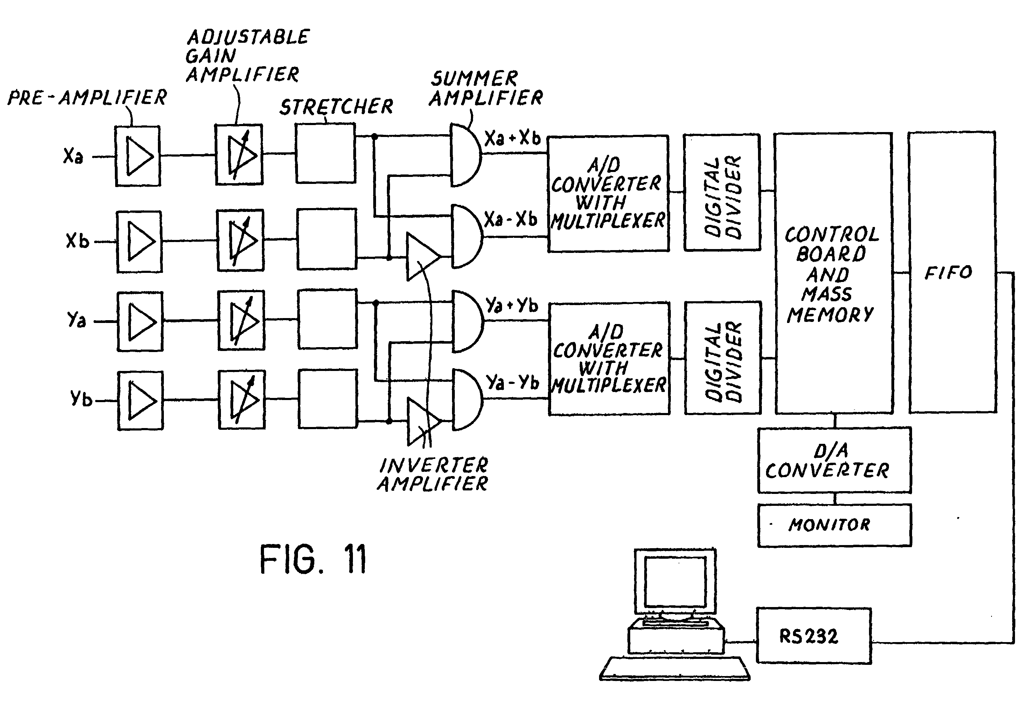

Aiming at eliminating any noise induced by the photo-multiplier in the anode wires, a threshold read system can be additionally provided, as it is shown in Figure 11.

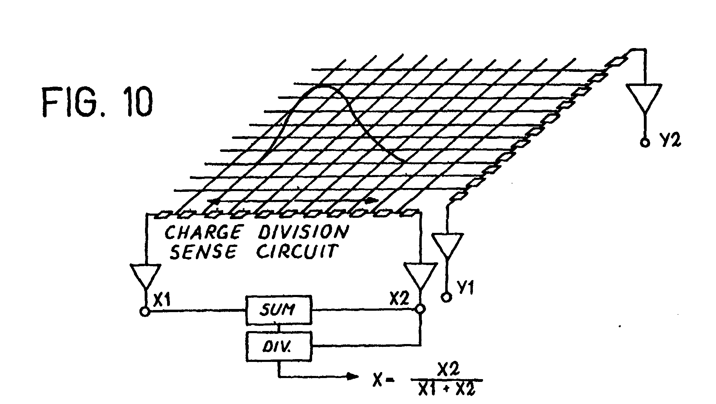

In a second embodiment, the reading and first processing operations of the charge signals collected by the photo-multiplier anode wires can be carried out by means of resistive chains. In such an arrangement, as shown in Figure 10, the ends of the two illustrated resistive chains generate four charge pulses Xa, Xb, Ya and Yb. As it is shown in the block diagram, the signals are coupled to a chain comprising a pre-amplifier and a variable gain amplifier in order to equalise the concerned signals. The sampling and synchronisation operations of the analog pulses are then carried out by a stretcher sampler module and, when information connected with each iteration position is to be obtained, the following functions:

$$X=(Xa-Xb)/(Xa+Xb)$$

$$Y=(Ya-Yb)/(Ya+Yb)$$

are to be carried out by means of adder and logic inverter devices. The addends of the division are then switched by an analog multiplexer device and are converted by a 10 bit analog-digital converter to realise the division operation. At this point, as in the previous embodiment, the result is then sent to a control station and to a mass memory, which re-processes and re-organises them into strings. Lastly, the concerned data, upon passing through a FIFO memory register, are transmitted to a Personal Computer and to a computer station by means of a connection serial interface.

Those skilled in the art will appreciate that a standard radiographic cassette can be housed upon the surface of the sensitive head section so as to perform overlapping diagnoses, comprising radiographic analysis and, upon removing the standard cassette, scintigraphic analysis, in time succession.

The preferred embodiments of this invention have been described and a number of variations have been suggested hereinbefore, but it should expressly be understood that those skilled in the art can make other variations and changes, without so departing from the scope thereof, as defined in the annexed claims.

**Claims**

**1.** An apparatus for carrying out scintigraphic mammography with submillimetric spatial resolution by detecting gamma radiations with 30 KeV to 2 MeV energy, comprising, in a sensitive head section (10) with a box shaped shielding container (16):

- an apertured collimator (11) for collimating the gamma radiation emissions from the body organ to be investigated;
- a scintillating crystal structure (12) for converting the gamma radiations emitted from the concerned body organ into light radiations;
- a photo-detector assembly (13) to detect the light radiations emitted from said scintillating crystals and to generate signals proportional to the received light radiations and

- a hardware assembly (15) to perform conversion and integration of all signals generated by said photo-detector assembly from an analog to a digital format as well as their amplification in order to subsequently couple them to an electronic processor which processes and displays them on a suitable monitor in the form of an image of the concerned organ,

   **characterized in that**:

- said collimator (11) is of a high atomic number material selected among lead, tungsten and like and it has apertures having a diameter in the range between 0.5 and 1.5 mm, particularly 0.6 mm, as well as aperture separation baffles with a thickness in the range between 0.05 and 0.5 mm, particularly 0.1 mm;
- said scintillating crystal structure (12) comprises a matrix of minicrystals made of material selected among YAP : Ce, Nal, Csl, BGO and like, particularly YAP : Ce, they have a cross-section dimension in the range between 0.3 x 0.3 and 2 x 2 mm, particularly 1 x 1 mm, with a thickness in the range between 0.5 and 20 mm, particularly 10 mm; the individual minicrystal of said matrix are optically separated from one another and any dead area between the individual minicrystals is in the range between 2 and 0.5 μm;
- said photo-detectors (13) are position-sensitive photo-multipliers having a crossed wire anode with a pitch of 3.75 mm or less or position-sensitive solid state detectors,
- a pressure member (17) is provided to compress the body organ to be investigated against the collimator (11) arranged at the lower face of the head section (10).

2. A scintigraphic mammograph according to claim 1 **characterised in that** a bundle of optical fiber light guides is provided to optically couple said scintillating minicrystals (12) to said photo-detector assembly (13).

3. A scintigraphic mammograph according to claim 1 **characterised in that** said scintillating crystal structure (12) comprises a layered plane structure including planar scintillating crystals and fluorescent fibers and the scintillation light is read in orthogonal and parallel directions with respect to the crystals by means of photo-multiplier devices in order to receive and read both the light emitted from the crystals and the light emitted from the fibers.

4. A scintigraphic mammograph according to claim 2 **characterised in that** said optical fiber light guides are made of plastic material selected among PMMA, PS, PC and like or of non-plastic material selected among glass, silica, quartz and like.

5. A scintigraphic mammograph according to claims 2 and 4 **characterised in that** said optical fibers are optically separated from one another by means of a reflecting coating deposited upon their interface surface, they have square or hexagonal cross section shape and they are assembled into a bundle having at its base a cross section area equal to the total surface area of said minicrystal matrix, without dead areas forming between the fibers.

6. A scintigraphic mammograph according to claims 1 and 5 **characterised in that** the coupling surfaces between said crystals and said optical fibers are curved, in particular with spherical, paraboloidal, ellipsoidal curvature or they are pyramidal.

7. A scintigraphic mammograph according to claim 5 **characterised in that** said optical fiber bundle is divided, at its end opposite to its base, into a number of sub-bundles, each having a cross section similar, in respect of shape and dimensions, to the active window of a plurality of photo-multipliers or solid state detectors used therein, and a resulting cumulative cross section area equal to the said base area.

8. A scintigraphic mammograph according to claim 5 **characterised in that** said optical fiber bundle has, at its end opposite to its base, a cross section area smaller than said base area, so as to be coupled to a single photo-multiplier or solid state detector and additionally it has a frusto-conical or frusto-pyramidal shape.

9. A scintigraphic mammograph according to claims 7 and 8, **characterised in that** the geometric shape of the top is different from the geometric shape of the base of said optical fiber bundle.

10. A scintigraphic mammograph according to claim 1 **characterised in that** said position sensitive photo-multipliers are responsive to signals emitted from the individual fibers associated with each individual dynode, so as to realise a high spatial resolution.

11. A scintigraphic mammograph according to claim 1 **characterised in that** said solid state detectors are silicon or germanium photo-diodes with a pixel figure in the range between 0.2 x 0.2 mm and 1 x 1 mm, particularly 0.5 x 0.5 mm, adapted to collect and read light photons, to locate their positions and to detect their energy absorbed by each scintillating minicrystal in its interaction with the gamma rays.

**12.** A scintigraphic mammograph according to claim 3 **characterised in that** the crystals forming said structure are of a material selected among YAP : Ce, NaI, CsI, BGO and like, particularly NaI (TI) and said fluorescent fibers are made of a plastic material selected among PMMA, PS, PC and like.

**13.** A scintigraphic mammograph according to claims 3 and 12 **characterised in that** said crystal have a thickness in the range between 0.2 mm and 10 mm, particularly 0.5 mm, and a surface area in the range between 50 x 50 sq. mm and 250 x 250 sq. mm, particularly 200 x 200 sq. mm.

**14.** A scintigraphic mammograph according to claim 1, **characterized in that** said scintillating crystal structure (12) comprises a number of scintillating tiles, arranged side-by-side, having a square cross-section shape with dimensions in the range between 0.3 mm x 0.3 mm and 50 mm x 50 mm or a rectangular cross section shape with short side dimensions in the range between 1 mm and 40 mm and long side dimensions in the range up to 250 mm or any other shape, and **in that** said tiles can be optically separated, the dead area between individual crystals being in the range between 2 $\mu$m and 2 mm, particularly 10 $\mu$m, and the thickness of the tiles being in the range between 0.2 mm and 10 mm, particularly 0.5 mm.

**15.** A scintigraphic mammograph according to claim 3 **characterised in that** two fluorescent fiber layers coupled to the two surfaces of each scintillating crystal are arranged in orthogonal directions or in directions forming any angle with respect to one another, in order to obtain two-dimensional information.

**16.** A scintigraphic mammograph according to claim 3 **characterised in that** said fluorescent fibers are made of a non-plastic material selected among glass, silica, quartz and like and they are optically separated from one another by means of a reflecting coating deposited upon their interface surfaces, in order to prevent the light from passing from a fiber to the adjacent ones.

**17.** A scintigraphic mammograph according to claims 1, 12 and 14 **characterised in that** a thin multilayer is deposited upon the surface of the crystals so as to realise an anti-reflection surface, in order to increase the crystal scintillation percentage exiting therefrom in a direction to said fluorescent fibers.

**18.** A scintigraphic mammograph according to claims 1 and 12-14 **characterised in that** the structure comprising overlapping planes of crystals and fibers is substituted by a structure comprising side-by-side arranged planes of crystals and possibly fibers arranged in a plane vertical or sloped with respect to the horizontal plane, the crystals and the possible fibers are read in vertical and horizontal directions by a photo-multiplier system or by a solid state CCD device assembly, and **in that** any dead areas formed by any fiber or any crystal containment material are eliminated by sloping the gamma-camera with respect to a vertical line or by successively displacing it for distances corresponding to fractions of the thickness of the fibers or of said containment material, with subsequent merge of the obtained images, the dimensions of the crystals being about 0.5 mm x 10 mm x 200 mm.

**19.** A scintigraphic mammograph according to claims 1 and 12 **characterised in that** the fiber layers arranged parallel to the crystals are substituted by optical fibers or lenses arranged in orthogonal direction with respect to the crystal layer and having a numeric aperture figure, namely the acceptance angle of the scintillator light, such that the light is enabled to pass only under determined angles or such that the light is oriented only in perpendicular direction with respect to the crystals.

**20.** A scintigraphic mammograph according to claims 1 and 12 **characterised in that** the surface of each planar crystal opposite to the X-ray entrance surface or any interface surface between crystals bears a thin multi-layer of a material having a controlled refraction index, so as to enable the scintillation light to emitted from the crystals under such angles as to illuminate a restricted area of the photo-cathode, the contact between successive planar crystals and the photo-multiplier assembly being continuous without any interposed fibers.

**21.** A scintigraphic mammograph according to claims 1 and 147 **characterised in that** a plane of microfibers of scintillating crystals is used and the light is directly conveyed to the photo-multiplier and/or by means of optical or fluorescent fibers, the individual crystals have dimensions in the range of 1 mm x 1 mm x 3 mm, the fibers can act as a means to convey the light to the photo-multiplier system, thereby forming variable cross section structures in order to overcome the dead areas of the photo-multiplier or they can form a plane overposed upon the crystal matrix so as to enable an additional useful reading in said dead areas of the photo-detectors.

**22.** A scintigraphic mammograph according to claim 1 **characterised in that** the surface of the mammograph is curved rather than planar, with cylindrical or paraboloidal curvature or it has an anatomic shape.

**23.** A scintigraphic mammograph according to claims 1 to 3 **characterised in that** the an amplification of scintillation light of the crystals is performed by means of the stimulated emission phenomenon realised by a pumping action of a laser or of a lamp.

**24.** A scintigraphic mammograph according to claims 1 to 3 **characterised in that** the apertures (11a) of the collimator (11) are not parallel but they form a convergent, divergent or focalising structure, or a pin-hole structure, they may be oblique or alternatively oblique in two directions, or oblique in a number of directions, in order to favour the elimination of said dead areas or to aid the evaluation of the depth of the tumor.

**25.** A scintigraphic mammograph according to claims 1 to 24 **characterised in that** optical fibers or tiles of plastic or non-plastic material are coupled to the lateral surfaces of said planar crystals or of said scintillating tiles, acting as light guides, in order to more easily locate the fluorescent fibers related to the scintillation area.

**26.** A scintigraphic mammograph according to claims 1-3 **characterised in that** said fluorescent fibers have their first ends covered by a reflecting material and their other ends coupled to a photo-multiplier anode.

**27.** A scintigraphic mammograph according to claims 1-3 **characterised in that** said solid state detectors are made by photo-diodes of silicon or other materials or by CCD cameras with pixel dimensions in the range between 12 x 12 μm and 2.54 x 2.54 mm, adapted to read light photons, in order to determine the position and the energy released by any scintillating crystal.

**28.** A scintigraphic mammograph according to anyone of the precedent claims, **characterised in that** it is mounted upon a movable mechanical support, adapted to move its head section and the pressure member along all three spatial co-ordinates in order to enable investigations to be carried out from any position in a 360° arc, as well as to transport the mammograph in order to analyse patients immobilised in beds.

**29.** A scintigraphic mammograph according to anyone of the precedent claims, **characterised in that** a two-dimensional image of the object to be investigated is obtained by means of reading operations carried out in angled directions, in particular in orthogonal directions of the fluorescent fibers.

**30.** A scintigraphic mammograph according to anyone of the precedent claims, **characterised in that** a two-dimensional image of the object to be investigate together with an evaluation of the depth or distance from the detector are obtained by means of a multi-plane or multi-layer structure according to anyone of the following mechanisms: a) on each plane, images are obtained consisting of the intersections of planes with a cone having the object being analysed at its apex; b) in view of the particular collimator used therein, particularly crossed or focalising collimators, the image is focalised upon one of the planes of the multi-plane structure; c) tomographic images are obtained by means of curved surfaces, particularly paraboloidal, cylindrical or anatomic surfaces, according to the mechanical characteristics of the plastic fibers; d) two images of any tumor are obtained by using oblique crossed collimators; e) the localisation direction of any tumor can be crossedly tracked by means of oblique crossed collimators and of a detector multi-plane structure.

**31.** A scintigraphic mammograph according to anyone of the precedent claims, **characterised in that** a standard radiographic cassette can be mounted upon the surface of said head section so as to enable overlapping diagnoses to be made, namely a radiographic analysis and, upon removing the standard cassette, a scintigraphic analysis, in time succession.

**32.** A scintigraphic mammograph according to claim 1 **characterised in that** said the hardware assembly for processing the signals coming from said photo-multiplier comprises a pre-amplification stage and a variable gain amplification stage, to the outputs of which stretcher circuits are coupled to further extend the signal and to apply them to analog multiplexer circuits, the outputs of said analog multiplexer circuits are coupled to analog-digital converter circuits and subsequently to a control and mass memory block, which organises the data into strings, each associated to an interaction event in the crystal, the output strings being then shifted in FIFO memory registers and then transmitted through a serial interface RS232 to a Personal Computer and to a computer station in order to carry out the necessary processing operations aimed at forming the image.

**33.** A scintigraphic mammograph according to claim 1 **characterised in that** said hardware assembly for processing the signals coming from the photo-multiplier comprises resistive chains for collecting the charges from the anode wires of the photo-multiplier and for generating four charge pulses (Xa, Xb, Ya, Yb) which are applied to respective chains comprising, in series connection, a pre-amplifier, a variable gain amplifier and a stretcher circuit, as well as adder and inverter circuits to produce sum and difference signals (Xa+Xb, Xa-Xb, Ya+Yb, Ya-Yb), then multiplexer and analog-digital converter circuits and subsequently divider circuits to calculate the functions

$$X = (Xa-Xb) / (Xa+Xb)$$

$$Y = (Ya-Yb) / (Ya+Yb),$$

the result of the division being applied to a control and mass memory block which processes and re-organises the data into strings and applies them through FIFO memory registers and a serial interface RS232 to a Personal Computer and to a computer station for performing the necessary processing operations in order to form the image.

**Patentansprüche**

1. Vorrichtung zur Durchführung einer szintigraphischen Mammographie mit einer raumlichen Auflösung im Submillimeterbereich durch Erfassung von Gemmastrahlungen mit einer Energie von 30 KeV bis 2MeV, enthaltend, in einer wärmeempfindlicken Haupt-Abteilung (10) mit schachtelförmigen Abschirmungsgehäuse (16),

   - einen gelochten Kollimator (11) für die Kollimation der Gammastrahlungsemissionen eines zu überprüfenden Körperorgans;
   - eine Szintillazion- Kristallinstruktur (12) zur Umwandlung der vom jeweiligen Körperorgan ausgestrahlten Gammastrahlungen in Lichtstrahlungen;
   - eine Photo-Detektoranordnung (13) zur Erfassung der von vorgenannten Szintillazienskristallen asgestrahlten Lichtstrahlungen und zur Erzeugung von zu den erhaltenen Lichtstrahlungen proportienellen Signalen und
   - eine Hardware-Anordnung zur Durchfühung einer Umwandlung und Integration von allen durch die vorgenannte Photo-Detektoranordnung erzeugten Signalen vom analogischen zu digitalischen Format, sowie zur deren Verstärkung, um diese dann in einen elektronischen Prozessor einzukuppeln, der sie verarbeitet und auf einem angebrachten Monitor in Form eines Bildes des jeweiligen Organs sichtbar nacht, dadurch gelannzeichnet, dass
   - der vorgenannte Kollimator (11) aus einem Hochtomzahlwerkstoff, wie Blei, Tungsten u. degl. besteht und Öffnungen mit einem Durchmosser von 0,5 bis, 1,5 mm, insbesondere von 0,6 mm sowie Trennrippen fur die Öffnungen mit einer Dicke von 0,05 bis 0,5 mm, insbesonder 0,1 mm, hat;
   - die vorgenannte Szintillation-Krystallinstruktur (12) eine Minikristallinmatrix aus einem unter YAP:Ce, Naö, Csl, BGO und dergleichen augewählten Stoff, insbesondere YAP:Ce, mit einem Querschmitt zwischen 0,3 x 0,3 und 2 x 2 mm, insbesondere 1 x 1 mm und mit einer Dikke zwischen 0,5 und 20 mm, insbesondere 10 mm, wobei die Minikristalle der vorgenannten Matrix voneinander optisch getrennt sind und jede Todfläche zwischen den einzigen Minikristallen in einem Bereich von 2 bis 0,5 mm liegt;
   - die vorgenannten Photo-Detektor (13) lageemplindliche Photovervielfacher, die eine Kreuzdraht-Anode mit einer Steigung von 3,75 mm oder weiniger haben oder lageempfindliche Festkörper-Detektore sind;
   - ein Druckteil (17) vorgesehen ist, das zum Andrücken des zu untersuchenden Körperorgans an den an der unteren Fläche der Kopfabteilung (10) angrodneten Kollimator (11) dient.

2. Vorrichtung zur Durchführung einer szintigraphischen Mammographie nach Anspruch 1, **dadurch gekennzeichnet, dass** ein Bündel von lichtleitenden Fasern zur optischen Ankupplung der vorgenannten Scintillationsminikristallen (12) an die vorgenannte Photo-Detektoranordnung (13) vorgesehen ist.

3. Vorrichtung zur Durchführung einer szintigraphischen Mammographie nach Anspruch 1, **dadurch gekennzeichnet, dass** die vorgenannte Szistillation-Kristallinstruktur (12) eine geschichtete ebene Anordnung von Szintillationskristallen und fluoreszenten Fasern hat und die Szintillationslicht in senkrechten und parallelen Richtungen zu den Kristallen durch Photovervielfachervorrichtungen gelesen wird, um das von den Kristallen und von den Fasern ausgestrachlte Licht zu erhalten und lesen.

4. Vorrichtung zur Durchführung einer szintigraphischen Mammographie nach Anspruch 2, **dadurch gekennzeichnet, dass** die vorgenannten lichtleitenden Optikfasern aus einem Kunststoff, der zwischen PMMA, PS, PC u.drgl. ausgewählt ist oder aus einem nicht plastischen Stoff, der unten Glas, Silizium, Quartz und dergleichen ausgewählt ist, bestehen.

5. Vorrichtung zur Durchführung einer szintigraphischen Mammographie nach Anspruchs 2 und 4, **dadurch gekennzeichnet, dass** die vorgenannten Optikfasern voneinander durch eine auf deren angrenzenden Flächen angebrachte reflektierende Umhüllung abgetrennt sind, eimen rechteckigen oder seehswinkligen Querschitt haben und in ein Bündel zusammengefasst sind, das an seiner Basis eine Querschnitt aufweist, der der gesamten Oberfläche der vorgenannten Minikristallinen-Matrix, ohne den zwischen den Fibern gebildeten Todberichen, entspricht.

6. Vorrichtung zur Durchführung einer szintigraphischen Mammographie nach Anspruch 1 und 5, **dadurch gekennzeichnet, dass** die Kupplungsflächen zwischen den vorgenannten Krstallenengekümmt sind und zwar mit einer kugeligen, paboloidformigen, ellipsoidformigen Krümmung oder pyramideförmig sind.

7. Vorrichtung zur Durchführung einer szintigraphischen Mammographie nach Anspruch 5, **dadurch gekennzeichnet, dass** das Optikfiberbündel an seinem der Basis entgegengesetzten Ende in eine Anzahl von Unterbündeln unterteilt ist, von denen jedes einen Querschnitt, der grössen- und formmässig dem wirksamen Fenster einer Mehrzahl von in demselben angewandten Photo- Vervielfälchern oder Fastkörper detektoren entspricht und einen Summenquerschmitt hat, der eine der vorgenannten Besisfläche gleiche Fläche aufweist.

8. Vorrichtung zur Durchführung einer szintigraphischen Mammographie nach Anspruch 5, **dadurch gekennzeichnet, dass** des vorgenannte Optikfaser-Bündel, an seinem der Basis entgegensetzen Ende, eine Quischnittfläche hat, die kleiner als die vorgenannte Basis fläche ist, so dass sie an einen einzeluen Photovercielfältiger oder Festkörper-Detektor angekuppelt werden, ausserden wobei sie eine kegelstumpfige oder pyramidenstumpfige Form hat.

9. Vorrichtung zur Durchführung einer szintigraphischen Mammographie nach Anspruch 7 und 8, **dadurch gekennzeichnet, dass** die geometrische Form des Gipfels von derjenigen der Basis des vorgenennten Optikfaser-Bündel unterschiedlich ist.

10. Vorrichtung zur Durchführung einer szintigraphischen Mammographie nach Anspruch 1, **dadurch gekennzeichnet, dass** die vorgenannten lageempfindlichen Photovemielfacher auf die von den einzelnen an jede einzelne Dynode angekuppelten Fasern ausgestrahlte Signale empfindlich sind, so das eine hohe raumliche Auflösung erreicht wird.

11. Vorrichtung zur Durchführung einer szintigraphischen Mammographie nach Anspruch 1, **dadurch gekennzeichnet, dass** die vorgenannten Festkörper-Detektore aus Silizium- oder Germanium-Photodioden bestehen, mit einem Pixelgebilde in einem Bereich zwischen 0,2 x 0,2 mm und 1 x 1 mm, insbesondere 0,5 x 0,5 mm, die zum Autsammeln und Lesen der Lichtphotonen, zum Anzeigen deren Stellung und zum Erfassen deren vom jeden Swintillation-Minikristall bei Mitwirkung der Gammastrahlen aufgenommenen Energie.

12. Vorrichtung zur Durchführung einer szintigraphischen Mammographie nach Anspruch 3, **dadurch gekennzeichnet, dass** die die vorgenannte Struktur bildenden Kristalle aus unter YAP:CE, NaI, CsI, BGO und dgl., insbesondare NaI (TI) ausgewählten Stoffen bestehen und die vorgenannter fluoreszierenten Fasern aus einem unter PMMA, PS, PC und dgl. Kunststoffausgewähtten Stoff hergestellt sind.

13. Vorrichtung zur Durchführung einer szintigraphischen Mammographie nach Anspruch 3 und 12, **dadurch gekennzeichnet, dass** die vorgenannten Kristalle eine Stärke zwischen 0,2 und 10 mm, insbesondere 0,5 mm und eine Oberfläche im Bereich zwischen 50 x 50 und 250 x 250 mm$^2$ insbesondere 200 x 200 mm$^2$ haben.

14. Vorrichtung zur Durchführung einer szintigraphischen Mammographie nach Anspruch 1, **dadurch gekennzeichnet, dass** die vorgenannte Scintillation-Krystallinstruktur (12) eine Anzahl von aneinander gereihten Szintillationsziegeln aufweist, die einen quadratischen Querschnitt der Grösse zwischen 0,3 x 0,3 mm und 50 x 50 mm oder einen rechteckigen Querschnitt, dessen kürzere Seit von 1 bis 40 und dessen längere Seite bis 250 mm lang ist, oder eine andere Querschnittsform haben und dass die genannten ziegeln optisch separiert werden können, wobei der Todbereich zwischen den einzelnen Ziegelu eine Grösse von 2 μm und 2 mm, insbesondere 10 μm hat und die Ziegelstärke von 0,2 mm bis 10 mm, insbesondere 0,5 mm beträgt.

15. Vorrichtung zur Durchführung einer szintigraphischen Mammographie nach Anspruch 3, **dadurch gekennzeichnet, dass** zwei fluoriszierende an die zwei Oberflächen eines jeden Szintillationskristalls angekuppelten Faser in orthogonalen oder in miteinander einen jeden Winkel bildenden Richtungen angeordnet sind, um eine zweidimensionele Information zu erhalten.

16. Vorrichtung zur Durchführung einer szintigraphischen Mammographie nach Anspruch 3, **dadurch gekennzeichnet, dass** die vorgenannten fluoriszierenden Faser aus eimem unter Glas, Silizium, Quartz und drgl. ausgewählten nicht plastischen Stoff bestehen und durch eine reflektierende, auf deren angrengenden Flächen abgelagerte Umhüllung voneinander optisch getrennt sind, um zu verhindern, dass das Licht von einem zum anderen Faser übergeht.

**17.** Vorrichtung zur Durchführung einer szintigraphischen Mammographie nach den Ansprünchen 1, 12 und 14, **dadurch gekennzeichnet, dass** eine düne mehrschichtige Lage auf der Oberfläche der Kristalle abgelargert ist, um so eine vergütete Oberfläche zu bilden und die Prozentanzahl der Kristallszintillation in Richtung der genannten fluoreszierenden Faser zu erniedrigen.

**18.** Vorrichtung zur Durchführung einer szintigraphischen Mammographie nach den Ansprünchen 1, 12 und 14, **dadurch gekennzeichnet, dass** die übereinander liegende Schichten von Kristallen und Fasern enthaltende Struktur durch eine aus beinander liegenden Kristallinschichten und gegebenfalls aus in einer gegenüber der waagerechen Ebene senkrechten oder geneigten Ebene ligenden Fasern bestehende Struktur ersetzt ist, wobei die Kristalle und Faser durch eine Photovervielfalchungsanordnung oder durch Festköper CCD - Vorrichtung in senkrechter und waagerechter Richtung gelesen werden, und dass alle aus einem jeden Faser und Kristalle enthaltenden Stoff bestehendeTodbereiche durch Neigung der Gamma-Kamera gegenüber einer senkrechten Linie oder durch nachfolgende Verstellung derselben um die den Bruchteilen der Faserstärke oder des genannten Stoffes entsprechenden Strecken beseitigt werden, wodurch die erhaltenen Bilder sichtbar werden und wobei die Grösse von Kristallen etwa 0,5 mm x 10 mm x 200 mm ist.

**19.** Vorrichtung zur Durchführung einer szintigraphischen Mammography nach Ansprunch 1 und 12 **dadurch gekennzeichnet, dass** die parallel zu den Kristallen angeordneten Faser durch optische Faser oder Linsen ersetzt werden, die gegenüber den Kristallinsohichten senkrecht angeordnet sind und eine numerische Aperturzahl und zwar einen Aufnahmewinkel des Szintillationslichtes hat, so dass das Licht nur mit vorbestimmten Winkeln durchgehen kann oder so dass das Licht nur in waagerechter Richtung zu den Kristallen gerichlet ist.

**20.** Vorrichtung zur Durchführung einer szintigraphischen Mammographie nach Ansprunch 1 und 12, **dadurch gekennzeichnet, dass** die der Eingangsfläche oder einer jeden Fläche zwischen den Kristallen gegenüberstehende Oberfläche eines jeden ebenen Kristalls eine mehrschichtige Lage aus einem einen geprüften Lichntbrechugs index habenden Staff trägt, so dass das Szintillationslicht von den Kristallen in solchem Winkel ausgestrahlt wird, dass eine beschränkte Fläche der Photo-kathode belichtet wird, wobei die Berührung zwischen den nacheinander folgenden ebenen Kristtallen und der Photo-Vervielfachungs anordung, ohne zwischenliegende Fiber, ununterbrochen ist.

**21.** Vorrichtung zur Durchführung einer szintigraphischen Mammographie nach Ansprunch 1 und 14, **dadurch gekennzeichnet, dass** eine Schicht von Mikrofasern aus Szintillationskristallen angewandt wird und das Licht direkt oder vermittels der fluoreszierenden oder optischen Fiber zum Photo-Vervielfältiger geführt wird, die einzelnen Kristalle eine Grösse von 1x1x3mm haben, die Faser als ein Mittel zur Führung des Lichtes zum Photo-Vervielfältigungstem wirken können, wodurch Gebilden mit veränderlichen Querschnitten gebildet werden, um Todbereiche des Photo-VervielfAltigers zu vermeiden, oder sie eine über die Kristall-Matrix angeordnete Ebene bilden können, so dass eine weitere nützliche Lesemöglichkeit in den vorgenannten Todbereichen des Photodetektors geschaffen wird.

**22.** Vorrichtung zur Durchführung einer szintigraphischen Mammographie nach Ansprunch 1, **dadurch gekennzeichnet, dass** die Fläche der Mammographievorrichtung nicht eben sonder gektümmt ist und zwar mit zylindrischer oder paraboloidischer Krümmung oder sie eine anatomische Form hat.

**23.** Vorrichtung zur Durchführung einer szintigraphischen Mammographie nach Ansprunch 1 und 3, **dadurch gekennzeichnet, dass** eine Verstärkung des Szintillationseichtes der Kristalle durch eine angeregtes Austrahlungerscheinung vermittols einer Pumpenwirkung oder eines Lasers oder einer Lampe durchgeführt wird.

**24.** Vorrichtung zur Durchführung einer szintigraphischen Mammographie nach Ansprunch 1 und 3, **dadurch gekennzeichnet, dass** die Öffnungen (11a) des Kollimators (11) nicht parallel sind, sonderkönnen eine konvergente, divergente oder fokussierende Struktur oder eine Pin-Hole-Struktur bilden können, sie können schräg oder in einer Anzahl von Richtungen schräg verlaufen, un so die Beseitigung der genannten Todbereiche oder die Bewertung der Tumortiefe zu erleichtern.

**25.** Vorrichtung zur Durchführung einer szintigraphischen Mammographie nach Ansprunch 1 und 24, **dadurch gekennzeichnet, dass** die Optikfaser oder ziegel aus Kunst-oder Naturstoff an die Seitenfläche der vorgenannten ebenen Kristalle oder der vorgenannten als Lichtführungen wirkenden, Szintillationsziegel angekuppelt sind, um so die fluoreszierenden Faser gegenüber der Szintillationsfläche leichter anzuordnen.

**26.** Vorrichtung zur Durchführung einer szintigraphischen Mammographie nach Ansprunch 1 und 3, **dadurch gekennzeichnet, dass** ein Ende der genannten fluoreszierenden Faser mit einem lichtabweissenden Stoff bedeckt ist und das andere Ende en eine Anode des Photo-Vervielfachers angekuppelt ist.

27. Vorrichtung zur Durchführung einer szintigraphischen Mammographie nach Ansprunch 1 und 3, **dadurch gekennzeichnet, dass** die vorgenannten Festkorper-Detektore aus Photo-Dioden aus Silzium oder einem anderen Stoff oder aus CCD - Kamera mit Pixel-Mass im Bereich zwischen 12x12zum und 2,54x2,54 mm bestehen, die zur Bestimmung der Lage und der durch einen jeden Szintllationskristall ausgestrahlten Energie angebracht sind.

28. Vorrichtung zur Durchführung einer szintigraphischen Mammographie nach je einem der vorhergehenden Ansprüche, **dadurch gekennzeichnet, dass** sie auf einen verstellbaren mechanischen Lager montiert ist, der seine Hauptabteilung und das Druckglied in allen drei raumlichen Koordinaten zu verstellen vermag, um so die durchzuführende Untersuchung von jeder Stellung aus in einem 360° Bogen bereich sowieden Trasport der Mammographie-Vorrichtung für die Untersuchung von im Bett immobilisierten Patienten zu ermöglichen.

29. Vorrichtung zur Durchführung einer szintigraphischen Mammographie nach je einen der vorhergehenden Ansprüche, dadurch gekennsizeichnet, dass ein zweidimensionales Bild des zu untersuchenden Organs durch Lesen in Winkelrichtungen, insbesondere in senkrechten Richtungen der fluoreszierenden Faser erhalten wird.

30. Vorrichtung zur Durchführung einer szintigraphischen Mammographie nach je einem der vorhergehenden Ansprüche, **dadurch gekennzeichnet, dass** ein zweidimensionales Bild des zu untersuchenden Organs zusammen mit der Bewertung der Tiefe oder des Abtandes vom Detektor vermittels einer mehrschichtigen Struktur gemäss einer jeden der folgenden Vorgehensweisen erhalten werden: a) auf jeder Ebene Bilder erhalten werden, die aus Überschneidungen von Ebenen mit einem Kegel, der das zu untersuchenden Organ auf seinem Gipfet hat; b) in Angesicht des besonderen Kollimators, der hier gebraucht wird, insbesondere des gekreuzten oder Fokusierenden Kollimators, das Bild auf der einem Ebene der mehrschichtigen Strucktur fokusiert ist; c) tomographidische Bilder werden durch gekrümmte Flächen, insbesondere durch paraboloiscke, zylindrische oder anatomische Fläche, entsprechend der mechanischen Merkmale der Kunststoffaser erhalten; d) zwei Bilder eines Tumors werden dank des angewandten gekreuzten Kollimators erhalten; e) die Lokalisierungs-richtung eines Tumors kann kreuzweise vermittels der gekreuzten geneigten Kollimatoren und der mehrschichtigen Struktur des Detektors angezeigt werden.

31. Vorrichtung zur Durchführung einer szintigraphischen Mammographie nach je einem der vorhergehenden Ansprüche, **dadurch gekennzeichnet, dass** eine radiographische Standardkassette auf der Oberfläche der vorgenannten Hauptabteilung angeordnet wird, um so die Durchfürung von überlagerten Diagnosen, und zwar radiographischen Analysen und bei Entfernung der Standarkassette, eine szintigraphische Analyse zu gestatten.

32. Vorrichtung zur Durchführung einer szintigraphischen Mammographie nach Ansprunch 1, **dadurch gekennzeichnet, dass** die vorgenannte Hardware-Anordnung zur Verarbeitung der vum vorgenannten Fotovervielfacher ankommenden Signalen eine Vorverstärtungs-stufe und eine veränderliche Stromverstärkungs stufe enthält en deren Ausgänge Erweiterungsschaltungen zur weiteren Erstrekkung der signale und zur Ansterurung derselben an die analogischen Multiplexerschaltungen angeschlossen sind, deren Ausgänge an Analog-Digital-Umsetzerschaltungen angekupptelt sind, und dann an einen Steuer-und Speicherblock, der die Daten in Zeichenketten einordnet, von denen jede einem Wechselwirkunserreignis im Kristall zugeordnet ist, wobei die Ausgangzeichenketten dann in FIFO-Speicherregister verstellt und vermittals einer seriellen Schmitttstelle an einem Personal Computer und eine Computerstelle weitergeleitet werden, um so die notwendigen Verarbeitungsoperationen zur Erhaltung der Bilder durchzuführen.

33. Vorrichtung zur Durchführung einer szintigraphischen Mammographie nach Ansprunch 1, **dadurch gekennzeichnet, dass** die vorgenannte Hardware-Anordnung zur Verarbeitung der von Fotovervielfacher ankommenden Signale, Widerstandsketten zur Erfassung von Ladungen aus den Anodedrähten des Fotovervielfachers und zur Erzeugung von Landungsimpulsen (Xa, Xb, Ya, Yb) enthält, die den entsprechenden Ketten zugeführliche Stromverstärker und in Reiheschaltungeine Erwiterungsscheltung, sowie Addition und Wenderschaltungen zur Erhaltung von Addition-und Subtraktionsignalen (Xa+Xb, Xa-Xb; ya+Yb, Ya-Yb) und Multiplexer und Analog-Digital-Unsetzerschaltungen und Teilerschaltungen zur Errechnung der Funktionen

$$X=(Xa-Xb) / (Xa+Xb)$$

$$Y=(Ya-Yb) / (Ya+Yb)$$

enthält, wobei das Ergebnis der Teilung einem Steuer-und Massenspeicher-Block zugeführt wird, der die Daten verarbeitet und in Zeichenketten einordnet und diese vermittels FIFO Speicherregister und einer seriellen Schnittstelle RS232 und einem Personal Computer und einer Computerstation zur Durchführung die notwendigen Verarbeitungsoperationen zuführt, um so das Bild zu erhalten.

## Revendications

1.  Appareil pour exécuter des mammographies scintigraphique à résolution spatiale sous-millimetrique par détection des radiations gamma à une énergie de 30 KeV à 2 MeV, comprenant dans une section principale sensitive (10) avec un récipient de masquage à forme de boit (16):

    -   un collimateur (11) ayanat overtures pour viser les émissions de rayonnements gamma de l'organ corporel;
    -   une structure cristalline de scintillation (12) pour transformer les rayonnements gamma, émis de l'organ du corps en question, en rayonnements lumineux;
    -   un assemblage photo-détecteur (13) pour détecter les rayonnements lumineux émis desdits cristaux de scintillation et pour engendrer des signaux proportionellement aux rayonnements lumineux reçus et
    -   un assemblage de hardware (15) pour réaliser la conversion et l'intégration des tous les signaux, engendrés par ledit assemblage photodétecteur, u format analogique au format digitale, ainsi que l'amplification des mêmes afin de coupler lesdits signaux à un processeur électronique, qui élabore et visualise les mêmes sur un écran de contrôle adéquat sons forme d'une image de l'organ en question;
    -   ledit **caractérisé en ce que** ledit collimateur (11) est fait d'un matériau ayant un nombre atomique élevé, choisi entre plomb, tungstene et similaires et il a des ouvertures presentant un diamètre entre 0,5 et 1,5 mm, particulièrement 0,6 mm, ainsi que des écrans de séparation d'une ouvertures à un épaisseur entre 0,05 et 0,5 mm, particulièrement 0,1 mm;
    -   ladite structure cristalline de scintillation (12) comprend une matrice de mini-cristaux faits d'un matériau choisi entre YAP: Ce, Nal, Csl, BGO et similares, particulièrement YAP: Ce, ils ont une dimension de section trasversale entre 0,3 X 0,3 et 2 X 2 mm, particulièrement 1 X 1 mm, et un épaisseur entre 0,5 et 20 mm, particulièrement 10 mm; les minicristaux de ladite matrice étant optiquement separés l'un de l'autre et chaque aire morte entre les minicristaux individuelement varie entre 2 et 0,5 mm;

    -   lesdits photo-détecteurs (13) sont des photomultiplicateurs sensibles à la position, ayant un anode à fils croisés ayant un pas de 3,75 mm ou moins, ou des détecteurs à l'état solide sensibles à la position;
    -   un élément de pression (17) est prévu pour comprimer l'organ du corps à examiner contre le collimateur (11) placé sur la face inférieure de la section principale (10).

2.  Appareil de mammographie scintigraphique selon la revendication 1, caracterérisé en ce qu' un assemblage de guides de lumière à fibres optiques est prévu pour coupler optiquement lesdits miniricstaux à scintillation audit ensemble de photo-détecteurs (13).

3.  Appareil de mammographie scintigraphique selon la revendication 1, caracterérisé en ce que ladite structure cristalline à scintillation (12) comprend une structure plate stratifiée ayant des cristaux plats à scintillation et des fibres fluorescents et la lumière de scintillation est lue dans les directions orthogonales est parallèles par rapport aux cristaux au moyens des dispositifs photomultiplicateurs afin de recevoir et lire la lumière émise des cristaux et la lumière émise des fibres.

4.  Appareil de mammographie scintigraphique selon la revendication 2, caracterérisé en ce que lesdites guides à fibres optiques sont faites de matériaux plastiques choisis entre PMMA, PS, PC et similaires ou de matériels non plastiques choisis entre verre, silicium, quartz et similaires.

5.  Appareil de mammographie scintigraphique selon le revendications 2 et 4, caracterérisé en ce que lesdites fibres optiques sont optiquement séparées l'une de l'autre au moyen d'un revêtement réfléchissant déposé sur leur surface d'interface, elles ont une forme carrée ou hexagonale de leur section trasversale et sont assemblées en un faisceau ayant à sa base une section transversale égale à la surface totale de ladite matrice de minicristaux, sans compter les aires mortes formées entre les fibres.

6.  Appareil de mammographie scintigraphique selon la revendications 1 et 5, caracterérisé en ce que les surfaces de couplage entre lesdits cristaux et lesdites fibres optiques sont courbées, particulièrement à courbure sphérique, parabolique ellipsoidale ou elles sont pyramidales.

7.  Appareil de mammographie scintigraphique selon la revendication 5, caracterérisé en ce que ladit faisceau de fibres optiques est divisé à son extrémité opposé contraire à sa base, en un nombre des sous-faisceaux, chacun ayant une section trasversale similaire, concernant la forme et la dimension, à la fenêtre active d'une pluralité de photomultiplicateurs ou détecteur à l'état solide utilisée dans la même et la surface totale des sections trasversales est égale à la surface de base.

8. Appareil de mammographie scintigraphique selon la revendication 5, caracterérisé en ce que ledit faisceau de fibres optiques a, à son extrémité opposée à sa base, une aire plus petite que l'aire de la base, de façon à être accouplé à chaque photomultplicateur à l'état solide et, en outre, il a une forme de tronc de cône ou tronc de pyramide.

9. Appareil de mammographie scintigraphique selon la revendications 7 et 8, caracterérisé en ce que la forme géométrique du sommet est differente de la forme géométrique de la base dudit faisceau de fibres optiques.

10. Appareil de mammographie scintigraphique selon la revendication 1, caracterérisé en ce que lesdits photomultiplicateurs sensibles à la position sont apte à répondre au signaux émis de chaque fibre associée à chaque dynode, de façon à réaliser une haute résolution spatiale.

11. Appareil de mammographie scintigraphique selon la revendication 1, caracterérisé en ce que lesdits détecteurs à l'état solide sont des photo-diodes à silicium ou germanium avec une figure de pixel entre 0,2 X 0,2 mm et 1 X 1 mm, particulièrement 0,5 X 0,5 mm, apte à recevoir et lire les photons de la lumière, de façon à localiser leur positions et détecter leur énergie absorbée par chaque minicristal dans son interaction avec les rayons gamma.

12. Appareil de mammographie scintigraphique selon la revendication 3, **caractérisé en ce que** les cristaux formants ladite structure sont d'un matériau choisi entre YAP : CE, Nal, Csl, BGO et similaires, particulièrement Nal (TI) et lesdites fibres sont faites d'un matériau plastique choisi entre PMMA, PS, PC et similaires.

13. Appareil de mammographie scintigraphique selon les revendications 3 et 12, **caractérisé en ce que** ledits cristaux ont une épaisseur entre 0,2 mm et 10 mm, particulièrement 0,5 mm et une aire de surface entre 50 x 50 mm$^2$ et 250 x 250 mm$^2$, particulèrement 200 x 200 mm$^2$.

14. Appareil de mammographie scintigraphique selon la revendication 1, **caractérisé en ce que** ladite structure cristalline de sctinllation (12) comprend un nombre de briquettes, placées l'une à côte de l'autre, ayant une forme carré de section transversale et une dimension entre 0,3 mm x 0,3 mm et 50 mm x 50 mm ou une forme rectangulaire de section transversale ayant une dimension du côté court entre 1 mm et 40 mm et une dimension du côté long jusqu'à 250 mm, ou quelconque autre forme et **en ce que** ledsites briquettes peuvent être optiquement séparées, l'aire morte entre les cristaux étant comprise entre 2 µm et 2 mm, particulièrement 10 µm et l'épaisseur des briquettes étant entre 0,2 mm et 10 mm, particulièrement 0,5 mm.

15. Appareil de mammographie scintigraphique selon la revendication 3, **caractérisé en ce que** deux couches de fibres fluorescentes couplés aux deux surfaces de chaque cristal de scintillation, sont placées en directions orthogonales ou en directions formantes un angle quelconque entr' elles, de façon à obtenir des informations bidimensionnelles.

16. Appareil de mammographie scintigraphique selon la revendication 3, **caractérisé en ce que** lesdites fibres fluorescentes sont faites d'un matériau non plastique, choisi entre verre, silicium, quartz et similaires et sont séparées l'une de l'autre au moyen d'un revêtement réfléchissant déposé sur leur surfaces d'interface, de façon à empecher le passage de la lumière d'une fibre à la fibre adjacente.

17. Appareil de mammographie scintigraphique selon les revendications 1, 12 et 14, **caractérisé en ce que** une mince multicouche est deposée sur la surface des cristaux, de façon à réaliser une surface antiréfléchissante pour augmenter la pourcentage de scintillation des cristaux émise par la même dans une direction vers ledsites fibres fluorescèntes.

18. Appareil de mammographie scintigraphique selon les revendications 1 et 12 à 14, **caractérisé en ce que** la structure comprenant les plans superposés des cristaux et fibres est remplacée par une structure comprenant des plans de cristaux placées côté a côté et éventuellement des fibres placées dans un plan vertical ou incliné par rapport d au plan horizontal, les cristaux et les éventuelles fibres sont lus en directions verticale ou horizontale par un systeme de photomultiplicateurs ou par un ensemble de dispositives CCD à l'état solide et **en ce que** toutes les aires formées par des matériaux de confinement des cristaux et fibres sont éliminées en inclinant la chambre à rayons gamma par rapport a une ligne vertical ou en la déplaçant successivement d'un distance correspondant à une fraction d'épaisseur des fibres ou dudit matériau de confinement, avec la successive visualisation des images obtenues, les dimensions des cristaux étant proches de 0,5 mm $\times$ 10 mm $\times$ 200 mm.

19. Appareil de mammographie scintigraphique selon les revendications 1 et 12, **caractérisé en ce que** les couches de fibres, placées parallelement aux cristaux, sont remplacées par des fibres optiques ou lentilles placées en direction orthogonale par rapport aus couches de cristaux et ayant un nombre d'ouverture numérique, à savoir l'angle d'acceptation de la lumière du scintillateur, de façon que le lumière peut passer seulement sous des angles déterminés ou de façon que la lumière est orientée seulement en direction perpendiculaire par rapport aux cristaux.

20. Appareil de mammographie scintigraphique selon les revendications 1 et 12, **caractérisé en ce que** la surface de chaque cristal plat, contraire à la surface d'entrée des rayoins X, ou toutes les surfaces d'interface entre les cristaux, portent une mince multi-couche d'un matériel ayant un indice de réfraction controlé, de façon que la lumière de scintillation soit émise des cristaux sous un angle apte à illuminer un'aire limitée du photo-cathode, le contact entre les cristaux plats successifs et l'ensemble de photomultiplicateurs étant continu sans aucune interposition de fibres.

21. Appareil de mammographie scintigraphique selon les revendications 1 et 14, **caractérisé en ce qu'** on utilise un plan de microfibres des cristaux de scintillation et la lumière est irradiée au photo-multiplicateur directement ou an moyen des fibres optiques ou fluorescentes, chaque cristal a une dimension de 1 mm x 1 mm x 3 mm, les fibres peuvent agir comme un moyen de transmission de la lumière au systèm de photo-multiplicateurs, formant ainsi des structures à section transversale variable afin d' éliminer des aires mortes du photo-multiplicateur ou elles peuvent former un plan superposé sur la matrice cristalline, de façon à permettre une lecture additionelle utile dans leddites aires mortes du photo-détecteur.

22. Appareil de mammographie scintigraphique selon la revendication 1, **caractérisé en ce que** la surface du mammographe est corbe plutôt que plate, avec une corbure cylindrique, paraboïdale ou elle a une forme anatomique.

23. Appareil de mammographie scintigraphique selon les revendications 1 à 3, **caractérisé en ce qu'** une amplification de la lumière de scintllation des cristaux est réalisée au moyen d'un phénomène d'émission stimulé obtenu par une action de pompage d'un laser ou d'une lampe.

24. Appareil de mammographie scintigraphique selon les revendications 1 à 3, **caractérisé en ce que** les ouvertures (11a) du collimateur (11) ne sont pas paralleles, mais elles forment une structure convergente, divergente ou focalisante ou une structure de trou à pointeau, elles peuvent être obliques ou alternativement obliques en deux directions ou obliques en un nombre de directions, de façon à faciliter l'élimination desdites aires mortes ou à faciliter l'évaluation de la profondité de la tumeur.

25. Appareil de mammographie scintigraphique selon la revendications 3 et 12, **caractérisé en ce que** les fibres ou briquettes optiques de matériau plastique ou non-plastique sont couplées aux surfaces latérales desdits cristaux plats ou dedites briquettes scintillantes, en agissantes comme des guides de lumière, de façon à localiser plus facilement les fibres fluorescentes correspondantes à l'aire de scintillation.

26. Appareil de mammographie scintigraphique selon les revendications 1 à 3, **caractérisé en ce que** lesdites fibres fluorescentes ont leur extremités couvertes d'un matériau réflechissant et leur autres extremités couplées à une anode du photo-multiplicateur.

27. Appareil de mammographie scintigraphique selon les revendications 1 à 3, **caractérisé en ce que** lesdits détecteurs à l'état solide sont faits de silicium ou d'autres matériaux ou de caméras CCD avec dimension de pixel entre 12 x 12 $\mu$m et 2,54 x 2,54 mm, aptes à lire les photons de la lumière, de façon à déterminer la position et l'énergie irradiée par chaque cristal scintillant.

28. Appareil de mammographie scintigraphique selon quelconque des revendication précédentes, **caractérisé en ce qu'**il est monté sur un support mécanique mobile, apte à mouvoir sa section principale le long de toutes les trois coordonnées spatiales de façon à permettre le réalisation des recherches à partir de chaque position dans un arc de 360°, ainsi qu' à transporter le mammographe pour analiser des patients immobilisés au lit.

29. Appareil de mammographie scintigraphique selon une quelconque des revendications précédentes, **caractérisé en ce qu'** une image bidimensionelle de l'objet à examiner est obtenue au moyen d'un operation de lecture réalisée en directions angulaires, particulièrement en directions orthogonales des fibres fluorescentes.

30. Appareil de mammographie scintigraphique selon une quelconque des revendication précédentes, **caractérisé en ce qu'** une image bidimensionelle de l'object à examiner, conjointement à une évaluation de la profondité ou de la distance du détecteur, sont obtenues au moyen d'une structure à plusieurs plans ou couches selon un quelconque des mécanismes suivants: a) sur chaque plan on obtient des images, qui consistent en intersections des plans avec un cône ayant l'objet examiné a son sommet; b) compte tenu du collimateur spécial utilisé, particulièrement des collimateurs croisés ou focalisants, l'image est focalisé sur un des plans de la structure à plusieurs niveaux; c) des images tomographiques sont obtenues au moyen des surfaces courbées, particulièrement des surfaces paraboïdales, cylindriques ou anatomiques selon les caractéristiques mécaniques des fibres plastiques; d) deux images d'une tumeur sont obtenues en utilisant des collimateurs croisés; e) la direction de localisation d'une tumeur peut être tracée de façon croisée au moyen des collimateurs croisés obliquement et d'une structure à plusieurs plans de détection.

31. Appareil de mammographie scintigraphique selon quelconque des revendications précédentes, **caractérisé en ce qu'** une cassette radiographique standard peut être montée sur la surface de ladit section principale, de façon à permettre la réalisation des diagnoses superposées, à savoir une analyse radiographique et après la levée de la cassette standard, une analyse scintigraphique, en succession temporelle.

32. Appareil de mammographie scintigraphique selon la revendication 1, **caractérisé en ce que** ledit ensemble de hardware pour élaborer des signaux venants dudit photo-multiplicateur comprend une phase de pre-amplification et une phase d'amplification de gain variable, aux sortie duquel des circuits d'allongement sont couplés pour étendre ultérieurement le signale pour les appliquer aux circuits multiplexeurs analogiques, les sorties desdits circuits multiplexeurs étant couplée à des circuits convertisseurs analogiques digitaux et successivement à un bloc de contrôle et mémoire en masse, qui organise les données en chaînes de caractères, chacune associée à un événement d'interaction dans le cristal, les chaînes de caractères en sortie étant ensuite déplacées dans des registres de mémoire FIFO puis transmises an travers d'une interface sériele RS 232 à un ordinateur personnel et à une station de ordinateurs afin de réaliser les opérations d'élaboration nécessaires pour former l'image.

33. Appareil de mammographie scintigraphique selon la revendication 1, **caractérisé en ce que** ledit ensemble de hardware pour élaborer les signaux venant du photomultiplicateur comprend des chaînes de résistance pour absorber les charges des fils anodiques du photomultiplicateur et pour engendrer quatre implulsions de charge (Xa, Xb, Ya, Yb), qui sont appliquées aux chaînes correspondantes, comprenant, en connexion en série, un pre-amplificateur, un amplificateur de gain variable et un circuit d'allongement, ainsi que des circuits additionneurs et inverseurs pour produire des signaux de somme et de différence (Xx +Xb, Xa - Xb, Ya + Yb, Ya - Yb), puis des circuits multiplexeurs et convertisseurs analogique-digitaux et ensuite des circuits diviseurs pour calculer les fonctions:

$$X = (Xa\text{-}Xb) / (Xa\text{+}Xb)$$

$$Y = (Ya\text{-}Yb) / (Ya\text{+}Yb)$$

le résultat de la division étant appliqué à un bloc de contrôle et de mémoire de masse, qui élabore et réorganise les données en chaînes de caractères et les applique, à traver des registres de mémoire FIFO et une interface sériele RS 232, à un ordinateur personnel et à une station d'ordinateurs pour réaliser les opérations d'élaboration nécessaires pour former l'image.

FIG. 1

FIG. 2

FIG. 3

11

FIG. 4

FIG. 5

FIG. 6

**POWER SUPPLY AMPLIFIER CONVERTER**

**PRE-AMPLIFIER**

**PHOTO-MULTIPLIER**

**OPTICAL GUIDE**

**CRYSTAL**

**COLLIMATOR**

**EMITTER**

*Processing the digital signal and forming the image*

*Supplying the photo-multiplier, amplifying the received signal, converting the signal from analog to digital format*

*Pre-amplifying the analog signals*

*Converting ultraviolet-visible light to analog electrical signal*

*Conveying the ultraviolet-visible light from crystal to photo-multiplier*

*Converting gamma radiation to ultraviolet-visible light*

*Collimating gamma radiations to the crystal*

**FIG. 7**

28

EXTENSION
ALONG X-X
AXIS

Y

X-X AXIS
SWING

16

10

11

17

ROTATION
AROUND
X-X AXIS

Y-Y AXIS
SWING

Y

Z

ROTATION
AROUND
Z-Z AXIS

Z

FIG. 8

EP 0 829 022 B1

FIG. 9

EP 0 829 022 B1

FIG. 10

CHARGE DIVISION SENSE CIRCUIT

$$X = \frac{X2}{X1 + X2}$$

FIG. 11

FIG. 12

FIG. 13A

FIG. 13B

FIG. 14

FIG. 15